# EUROPEAN PATENT APPLICATION

(11) **EP 4 609 835 A2**
(43) Date of publication of application: **03.09.2025**
(21) Application number: 25189472.1
(22) Date of filing: 04.02.2021
(51) Int. Cl.: A61F 2/915

(54) **PROSTHETIC HEART VALVE LEAFLET COMMISSURE ASSEMBLIES AND METHODS**

(30) Priority: 06.02.2020 US 202062971011 P
(62) Divisional of application: 21708520.8
(71) Applicant: Edwards Lifesciences Corporation, Irvine, CA 92614 (US)
(72) Inventor: Nir, Noam, Irvine, CA 92614 (US); Sherman, Elena, Irvine, CA 92614 (US); Bukin, Michael, Irvine, CA 92614 (US); Levi, Tamir S., Irvine, CA 92614 (US); Yohanan, Ziv, Irvine, CA 92614 (US); Maimon, David, Irvine, CA 92614 (US)
(74) Representative: Eisenführ Speiser

(57) **Abstract**

An assembly and associated method for a commissure of a prosthetic heart valve is disclosed. As one example, a method includes forming a plurality of commissures with a plurality of leaflets of a prosthetic heart valve, wherein each commissure is formed by: folding each commissure tab of each leaflet into a series of overlapping layers, each folded commissure tab arranged outward of, in a lateral direction, a portion of a body of the leaflet that is directly connected to the commissure tab; pairing each folded commissure tab of each leaflet with a folded commissure tab of another leaflet; and securing at least a portion of the series of overlapping layers of each folded commissure tab in its folded configuration.

## Description

### CROSS REFERENCE TO RELATED APPLICATION

This application claims the benefit of U.S. Provisional Patent Application No. 62/971,011, filed February 6, 2020, which is incorporated herein by reference in its entirety.

### FIELD

The present disclosure relates to prosthetic heart valves, and to methods and assemblies for forming commissures with leaflets of such prosthetic heart valves.

### BACKGROUND

The human heart can suffer from various valvular diseases. These valvular diseases can result in significant malfunctioning of the heart and ultimately require repair of the native valve or replacement of the native valve with an artificial valve. There are a number of known repair devices (e.g., stents) and artificial valves, as well as a number of known methods of implanting these devices and valves in humans. Percutaneous and minimally-invasive surgical approaches are used in various procedures to deliver prosthetic medical devices to locations inside the body that are not readily accessible by surgery or where access without surgery is desirable. In one specific example, a prosthetic heart valve can be mounted in a crimped state on the distal end of a delivery device and advanced through the patient's vasculature (e.g., through a femoral artery and the aorta) until the prosthetic valve reaches the implantation site in the heart. The prosthetic valve is then expanded to its functional size, for example, by inflating a balloon on which the prosthetic valve is mounted, actuating a mechanical actuator that applies an expansion force to the prosthetic valve, or by deploying the prosthetic valve from a sheath of the delivery device so that the prosthetic valve can self-expand to its functional size.

Prosthetic valves that rely on a mechanical actuator for expansion can be referred to as "mechanically expandable" prosthetic heart valves. The actuator typically takes the form of pull cables, sutures, wires and/or shafts that are configured to transmit expansion forces from a handle of the delivery apparatus to the prosthetic valve.

Most expandable, transcatheter heart valves comprise a cylindrical metal frame or stent and prosthetic leaflets mounted inside the frame. The leaflets may be attached to the frame at commissure tabs (also referred to as leaflet tabs) of the leaflets. For example, a commissure may be formed by connecting the commissure tabs of two adjacent leaflets to one another, and in some embodiments, to a flexible sheet or attachment member configured to couple to a commissure support portion of the frame. The commissure can then be attached to the commissure support portion of the frame via a fastener, such as a suture. Typical commissures or commissure assemblies can be relatively complex and time consuming to form and suture to the commissure support portion of the frame, in part due to the numerous stitches that can be required. Further, these types of commissures and attachment methods to the commissure support portion can be subject to wear along the numerous stitches. For example, the mounted commissure can deteriorate due to displacement of the commissure out of its initial, secured position, including rotating around the commissure support portion and axially sliding up and down along the commissure support portion. Additionally, the sutures used to form the commissures and/or attach the commissures to the commissure support portion of the frame can be subject to wear when the sutures attach to and/or contact moving parts of the leaflets (e.g., the main body or working portion of the leaflet which moves during operation of the prosthetic heart valve), due to the sutures experiencing stress from holding a load of the prosthetic heart valve.

Accordingly, a need exists for improved prosthetic heart valve leaflet assemblies, and commissure formed with such leaflet assemblies, and methods for assembling the commissures to a frame of the prosthetic heart valve.

### SUMMARY

Described herein are embodiments of methods of assembling a prosthetic heart valve comprising a plurality of leaflets and prosthetic heart valves including a plurality of leaflets assembled together to form commissures configured to be coupled to a frame of the prosthetic heart valve. In some embodiments, the commissures may be formed by coupling a pair of adjacent commissure tabs of adjacent leaflets of the prosthetic heart valve to one another. The commissures may include a pair of two commissure tabs, each of the commissure tabs folded into a series of overlapping layers where majority portions of each overlapping layer are arranged in parallel with one another. One or more fasteners used to secure the two commissure tabs of the commissure to one another and/or to the commissure support portion or an attachment member configured to be coupled to the commissure support portion are separated from movable regions of bodies of the leaflets to which the commissure tabs of the pair of commissure tabs are connected by folds of the commissure tabs.

In one representative embodiment, a method of assembling a prosthetic heart valve comprising a plurality of leaflets can include forming a plurality of commissures with the plurality of leaflets, wherein each commissure is formed by: folding each commissure tab of each leaflet of the plurality of leaflets into a series of overlapping layers, wherein each leaflet includes two opposing commissure tabs arranged on opposite sides of a body of the leaflet, the body configured to move during operation of the prosthetic heart valve, wherein each folded commissure tab is arranged outward of, in a lateral direction arranged tangent to a circumference of the prosthetic heart valve, a portion of the body of the leaflet that is directly connected to the commissure tab; pairing each folded commissure tab of each leaflet with a folded commissure tab of another leaflet; and securing at least a portion of the series of overlapping layers of each folded commissure tab in its folded configuration; and for each commissure, attaching the commissure to a respective commissure support portion of a frame of the prosthetic heart valve, either directly or via an attachment member configured to be coupled to the commissure support portion, wherein the body of each leaflet is separated from the commissure support portion, the attachment member, and each fastener of one or more fasteners used for the securing and attaching via the folded commissure tab.

In another representative embodiment, a prosthetic heart valve can include: an annular frame comprising a plurality of commissure support portions; a plurality of attachment members, each secured to a corresponding support portion of the plurality of commissure support portions; and a plurality of leaflets, each leaflet comprising a body and two opposing commissure tabs arranged on opposite sides of the body, wherein each commissure tab is folded into a series of overlapping layers where majority portions of each overlapping layer are arranged in parallel with one another and overlap in a lateral direction, the lateral direction arranged tangent to a circumference of the annular frame, wherein each folded commissure tab is arranged adjacent to another folded commissure tab of an adjacent leaflet to form a pair of commissure tabs forming a commissure, and wherein each commissure is coupled to a corresponding attachment member of the plurality of attachment members; and wherein for each commissure: a first fastener is wrapped around at least a portion of the series of overlapping layers of both commissure tabs of the pair of commissure tabs of the commissure to secure the pair of commissure tabs together; a second fastener extends between an interior fold of a first folded commissure tab of the pair of commissure tabs and the corresponding attachment member to secure the first folded commissure tab to the attachment member; a third fastener extends between an interior fold of a second folded commissure tab of the pair of commissure tabs and the corresponding attachment member to secure the second folded commissure tab to the attachment member; and wherein each of the first, second, and third fasteners are separated from the bodies of the leaflets to which the commissure tabs of the pair of commissure tabs are connected by folds of the commissure tabs.

In another representative embodiment, a prosthetic heart valve can include: an annular frame comprising a plurality of commissure support portions; a plurality of attachment members, each secured to a corresponding support portion of the plurality of commissure support portions; and a plurality of leaflets, each leaflet comprising a body and two opposing commissure tabs arranged on opposite sides of the body, wherein each commissure tab is folded into a series of overlapping layers where majority portions of each overlapping layer are arranged in parallel with one another and overlap in a radial direction, relative to a central longitudinal axis of the prosthetic heart valve, wherein each folded commissure tab is arranged adjacent to another folded commissure tab of an adjacent leaflet to form a pair of commissure tabs forming a commissure, and wherein each commissure is coupled to a corresponding attachment member of the plurality of attachment members; and wherein for each commissure: a first fastener is wrapped around at least a portion of the series of overlapping layers of both commissure tabs of the pair of commissure tabs of the commissure to secure the pair of commissure tabs together; for each folded commissure tab of the pair of commissure tabs; a second fastener extends from a first interior fold of the folded commissure tab, through two overlapping layers of the series of overlapping layers, and to the corresponding attachment member to secure an inner portion, relative to a lateral direction arranged tangent to a circumference of the annular frame, of the folded commissure tab to the attachment member; and a third fastener extends from a second interior fold of the folded commissure tab, through one layer of the series of overlapping layers, and to the corresponding attachment member to secure an outer portion, relative to the lateral direction, of the folded commissure tab to the attachment member; and wherein each of the first, second, and third fasteners are separated from the bodies of the leaflets to which the commissure tabs of the pair of commissure tabs are connected by folds of the commissure tabs.

In another representative embodiment, a prosthetic heart valve can include: an annular frame comprising a plurality of commissure support portions; a plurality of attachment members, each secured to a corresponding support portion of the plurality of commissure support portions; a plurality of leaflets, each leaflet comprising a body and two opposing commissure tabs arranged on opposite sides of the body, wherein each commissure tab is folded into a series of overlapping layers with a terminal, free end of the commissure tab enclosed within the series of overlapping layers, wherein majority portions of each overlapping layer are arranged in parallel with one another and overlap in a radial direction, relative to a longitudinal axis of the prosthetic heart valve, wherein each folded commissure tab is arranged adjacent to another folded commissure tab of an adjacent leaflet to form a pair of commissure tabs forming a commissure, and wherein each commissure is coupled to a corresponding attachment member of the plurality of attachment members; and wherein, for each folded commissure tab of each commissure, a plurality of fasteners secure inner and outer portions, relative to a lateral direction arranged tangent to a circumference of the annular frame, of the folded commissure tab to the corresponding attachment member, wherein each of the plurality of fasteners are separated from the body of the leaflet to which the folded commissure tab is connected by folds of the folded commissure tab.

In another representative embodiment, a method of assembling a prosthetic heart valve comprising a plurality of leaflets can include: forming a plurality of commissures with the plurality of leaflets, wherein each commissure is formed by: folding each commissure tab of each leaflet of the plurality of leaflets into a series of overlapping layers that overlap in a lateral direction, the lateral direction arranged tangent to a circumference of the prosthetic heart valve, wherein each leaflet includes two opposing commissure tabs arranged on opposite sides of a body of the leaflet, the body configured to move during operation of the prosthetic heart valve, wherein each folded commissure tab is arranged outward of, in the lateral direction and a radial direction, a portion of the body of the leaflet that is directly connected to the commissure tab, the radial direction relative to a central longitudinal axis of the prosthetic heart valve; pairing each folded commissure tab of each leaflet with a folded commissure tab of another leaflet; and securing at least a portion of the series of overlapping layers of each folded commissure tab in its folded configuration; and for each commissure, attaching the commissure to a respective commissure support portion of a frame of the prosthetic heart valve, either directly or via an attachment member configured to be coupled to the commissure support portion, wherein the body of each leaflet is separated, in the radial direction, from the commissure support portion, the attachment member, and each fastener of one or more fasteners used for the securing and attaching via the folded commissure tab.

In another representative embodiment, a method of assembling a prosthetic heart valve comprising a plurality of leaflets can include: forming a plurality of commissures with the plurality of leaflets, wherein each commissure is formed by: folding each commissure tab of each leaflet of the plurality of leaflets into a series of overlapping layers that wind consecutively and overlap in a radial direction, relative to a central longitudinal axis of the prosthetic heart valve, wherein a free end of each commissure tab is arranged exterior to folds of the folded commissure tab, wherein each leaflet includes two opposing commissure tabs arranged on opposite sides of a body of the leaflet, the body configured to move during operation of the prosthetic heart valve, wherein each folded commissure tab is arranged outward of, in a lateral direction arranged tangent to a circumference of the prosthetic heart valve and the radial direction, a portion of the body of the leaflet that is directly connected to the commissure tab; pairing each folded commissure tab of each leaflet with a folded commissure tab of another leaflet; and securing at least a portion of the series of overlapping layers of each folded commissure tab in its folded configuration; and for each commissure, attaching the commissure to a respective commissure support portion of a frame of the prosthetic heart valve, either directly or via an attachment member configured to be coupled to the commissure support portion, wherein the body of each leaflet is separated from the commissure support portion, the attachment member, and each fastener of one or more fasteners used for the securing and attaching via the folded commissure tab.

In another representative embodiment, a method of assembling a prosthetic heart valve comprising a plurality of leaflets can include: forming a plurality of commissures with the plurality of leaflets, wherein each commissure is formed by: folding each commissure tab of each leaflet of the plurality of leaflets into a series of overlapping layers that overlap in a radial direction, relative to a central longitudinal axis of the prosthetic heart valve, by folding the commissure tab inward and over itself, such that an outermost, free end of the commissure tab is arranged within an interior of folds of the folded commissure tab, wherein each leaflet includes two opposing commissure tabs arranged on opposite sides of a body of the leaflet, the body configured to move during operation of the prosthetic heart valve, and wherein each folded commissure tab is arranged outward of, in a lateral direction arranged tangent to a circumference of the prosthetic heart valve and the radial direction, a portion of the body of the leaflet that is directly connected to the commissure tab; pairing each folded commissure tab of each leaflet with a folded commissure tab of another leaflet; and securing at least a portion of the series of overlapping layers of each folded commissure tab in its folded configuration; and for each commissure, attaching the commissure to a respective commissure support portion of a frame of the prosthetic heart valve, either directly or via an attachment member configured to be coupled to the commissure support portion, wherein the body of each leaflet is separated from the commissure support portion, the attachment member, and each fastener of one or more fasteners used for the securing and attaching via the folded commissure tab.

In another representative embodiment, a prosthetic heart valve can include: an annular frame comprising a plurality of commissure support portions; a plurality of attachment members, each secured to a corresponding support portion of the plurality of commissure support portions; a plurality of leaflets, each leaflet comprising a body and two opposing commissure tabs arranged on opposite sides of the body, wherein each commissure tab is folded into a series of overlapping layers including at least three layers, wherein majority portions of each overlapping layer are arranged in parallel with one another, wherein each folded commissure tab is arranged adjacent to another folded commissure tab of an adjacent leaflet to form a pair of commissure tabs forming a commissure, and wherein each commissure is coupled to a corresponding attachment member of the plurality of attachment members; and wherein, for each folded commissure tab of each commissure, a plurality of fasteners secure inner and outer portions, relative to a lateral direction arranged tangent to a circumference of the annular frame, of the folded commissure tab to the corresponding attachment member, wherein each of the plurality of fasteners are separated from the body of the leaflet to which the folded commissure tab is connected by folds of the folded commissure tab.

The foregoing and other objects, features, and advantages of the disclosed technology will become more apparent from the following detailed description, which proceeds with reference to the accompanying figures.

### BRIEF DESCRIPTION OF THE DRAWINGS

FIG. 1 is a perspective view of an exemplary embodiment of a prosthetic heart valve.
FIG. 2 is a perspective view of a frame for a prosthetic heart valve comprising three expansion and locking mechanisms, according to another embodiment.
FIG. 3 is a top plan view of the frame and expansion and locking mechanisms of FIG. 2.
FIG. 4A illustrates a first view of an example commissure of a prosthetic valve, in which the commissure is secured to a commissure support portion of a frame of the prosthetic valve.
FIG. 4B illustrates a second view of the assembled commissure of FIG. 4A.
FIG. 5A illustrates a cross-sectional view of the example assembled commissure of FIG. 4A in a first state.
FIG. 5B illustrates a cross-sectional view of the example assembled commissure of FIG. 4A in a second state.
FIG. 6 shows a first embodiment of a commissure assembly for a prosthetic heart valve.
FIG. 7 shows an embodiment of an attachment member that can be used in a commissure assembly to attach a commissure to a commissure support portion of a frame of the prosthetic heart valve, such as the commissure assembly of FIG. 6.
FIG. 8 shows the commissure assembly of FIG. 6, utilizing an alternate attachment member.
FIG. 9 shows a second embodiment of a commissure assembly for a prosthetic heart valve.
FIG. 10 shows the second embodiment of the commissure assembly which additionally includes fabric strips arranged between layers of the commissure.
FIG. 11 shows another embodiment of an attachment member, including a wire-form body, for attaching a commissure, such as the commissure shown in FIGS. 9 and/or 10, to a commissure support portion of a frame of the prosthetic heart valve.
FIG. 12 shows the commissure of FIG. 10 attached to the attachment member of FIG. 11.
FIG. 13 shows a third embodiment of a commissure assembly, in a first stage of assembly.
FIG. 14 shows the third embodiment of the commissure assembly of FIG. 13, in a second stage of assembly.
FIG. 15 is a flow chart of a method for assembling a prosthetic heart valve comprising a plurality of leaflets.
FIG. 16 is a side view of an embodiment of a transcatheter delivery apparatus for delivering a prosthetic heart valve to a target implantation site, with the prosthetic heart valve retained in a radially compressed state within a capsule of the delivery apparatus.
FIG. 17 is a side view of the transcatheter delivery apparatus of FIG. 16, with the capsule retracted to uncover the prosthetic heart valve.

### DETAILED DESCRIPTION

### General Considerations

For purposes of this description, certain aspects, advantages, and novel features of the embodiments of this disclosure are described herein. The disclosed methods, apparatus, and systems should not be construed as being limiting in any way. Instead, the present disclosure is directed toward all novel and nonobvious features and aspects of the various disclosed embodiments, alone and in various combinations and sub-combinations with one another. The methods, apparatus, and systems are not limited to any specific aspect or feature or combination thereof, nor do the disclosed embodiments require that any one or more specific advantages be present or problems be solved. The technologies from any example can be combined with the technologies described in any one or more of the other examples. In view of the many possible embodiments to which the principles of the disclosed technology may be applied, it should be recognized that the illustrated embodiments are only preferred examples and should not be taken as limiting the scope of the disclosed technology.

Although the operations of some of the disclosed embodiments are described in a particular, sequential order for convenient presentation, it should be understood that this manner of description encompasses rearrangement, unless a particular ordering is required by specific language set forth below. For example, operations described sequentially may in some cases be rearranged or performed concurrently. Moreover, for the sake of simplicity, the attached figures may not show the various ways in which the disclosed methods can be used in conjunction with other methods. Additionally, the description sometimes uses terms like "provide" or "achieve" to describe the disclosed methods. These terms are high-level abstractions of the actual operations that are performed. The actual operations that correspond to these terms may vary depending on the particular implementation and are readily discernible by one of ordinary skill in the art.

As used herein, with reference to the prosthetic heart valve and the delivery device or apparatus, "proximal" refers to a position, direction, or portion of a component that is closer to the user and a handle of the delivery apparatus that is outside the patient, while "distal" refers to a position, direction, or portion of a component that is further away from the user and the handle and closer to the implantation site. The terms "longitudinal" and "axial" refer to an axis extending in the proximal and distal directions, unless otherwise expressly defined. Further, the term "radial" refers to a direction that is arranged perpendicular to the axis and points along a radius from a center of an object (where the axis is positioned at the center, such as the longitudinal axis of the prosthetic valve).

As used in this application and in the claims, the singular forms "a," "an," and "the" include the plural forms unless the context clearly dictates otherwise. Additionally, the term "includes" means "comprises." Further, the terms "coupled" and "connected" generally mean electrically, electromagnetically, and/or physically (e.g., mechanically or chemically) coupled or linked and does not exclude the presence of intermediate elements between the coupled or associated items absent specific contrary language.

Directions and other relative references (e.g., inner, outer, upper, lower, etc.) may be used to facilitate discussion of the drawings and principles herein, but are not intended to be limiting. For example, certain terms may be used such as "inside," "outside,", "top," "down," "interior," "exterior," and the like. Such terms are used, where applicable, to provide some clarity of description when dealing with relative relationships, particularly with respect to the illustrated embodiments. Such terms are not, however, intended to imply absolute relationships, positions, and/or orientations. For example, with respect to an object, an "upper" part can become a "lower" part simply by turning the object over. Nevertheless, it is still the same part and the object remains the same. As used herein, "and/or" means "and" or "or," as well as "and" and "or."

For purposes of this description, certain aspects, advantages, and novel features of the embodiments of this disclosure are described herein. The described methods, systems, and apparatus should not be construed as limiting in any way. Instead, the present disclosure is directed toward all novel and non-obvious features and aspects of the various disclosed embodiments, alone and in various combinations and sub-combinations with one another. The disclosed methods, systems, and apparatus are not limited to any specific aspect, feature, or combination thereof, nor do the disclosed methods, systems, and apparatus require that any one or more specific advantages be present, or problems be solved.

Features, integers, characteristics, compounds, chemical moieties, or groups described in conjunction with a particular aspect, embodiment or example of the disclosure are to be understood to be applicable to any other aspect, embodiment or example described herein unless incompatible therewith. All of the features disclosed in this specification (including any accompanying claims, abstract, and drawings), and/or all of the steps of any method or process so disclosed, may be combined in any combination, except combinations where at least some of such features and/or steps are mutually exclusive. The disclosure is not restricted to the details of any foregoing embodiments. The disclosure extends to any novel one, or any novel combination, of the features disclosed in this specification (including any accompanying claims, abstract, and drawings), or to any novel one, or any novel combination, of the steps of any method or process so disclosed.

Although the operations of some of the disclosed methods are described in a particular, sequential order for convenient presentation, it should be understood that this manner of description encompasses rearrangement, unless a particular ordering is required by specific language set forth below. For example, operations described sequentially may in some cases be rearranged or performed concurrently. Moreover, for the sake of simplicity, the attached figures may not show the various ways in which the disclosed methods, systems, and apparatus can be used in conjunction with other systems, methods, and apparatus.

As used herein, the terms "a," "an," and "at least one" encompass one or more of the specified element. That is, if two of a particular element are present, one of these elements is also present and thus "an" element is present. The terms "a plurality of" and "plural" mean two or more of the specified element.

As used herein, the term "and/or" used between the last two of a list of elements means any one or more of the listed elements. For example, the phrase "A, B, and/or C" means "A," "B," "C," "A and B," "A and C," "B and C," or "A, B, and C."

As used herein, the term "coupled" generally means physically coupled or linked and does not exclude the presence of intermediate elements between the coupled items absent specific contrary language.

Directions and other relative references (e.g., inner, outer, upper, lower, etc.) may be used to facilitate discussion of the drawings and principles herein, but are not intended to be limiting. For example, certain terms may be used such as "inside," "outside,", "top," "down," "interior," "exterior," and the like. Such terms are used, where applicable, to provide some clarity of description when dealing with relative relationships, particularly with respect to the illustrated embodiments. Such terms are not, however, intended to imply absolute relationships, positions, and/or orientations. For example, with respect to an object, an "upper" part can become a "lower" part simply by turning the object over. Nevertheless, it is still the same part and the object remains the same. As used herein, "and/or" means "and" or "or," as well as "and" and "or."

In the context of the present application, the terms "lower" and "upper" are used interchangeably with the term's "inflow" and "outflow", respectively. Thus, for example, the lower end of the valve is its inflow end and the upper end of the valve is its outflow end.

As used herein, with reference to the prosthetic heart valve and the delivery apparatus, "proximal" refers to a position, direction, or portion of a component that is closer to the user and/or a handle of the delivery apparatus that is outside the patient, while "distal" refers to a position, direction, or portion of a component that is further away from the user and/or the handle of the delivery apparatus and closer to the implantation site. The terms "longitudinal" and "axial" refer to an axis extending in the proximal and distal directions, unless otherwise expressly defined. Further, the term "radial" refers to a direction that is arranged perpendicular to the axis and points along a radius from a center of an object (where the axis is positioned at the center, such as the longitudinal axis of the prosthetic valve).

In view of the many possible embodiments to which the principles of the disclosed invention may be applied, it should be recognized that the illustrated embodiments are only preferred examples of the invention and should not be taken as limiting the scope of the invention. Rather, the scope of the invention is defined by the following claims. We therefore claim as our invention all that comes within the scope and spirit of these claims.

### Examples of the Disclosed Technology

Described herein are examples of prosthetic heart valves, leaflet assemblies and commissures for prosthetic heart valves, and methods for assembling commissures of prosthetic heart valves. The prosthetic heart valves may include a frame and a plurality of leaflets attached to the frame via commissures formed by joining pairs of adjacent ends (e.g., commissure tabs) of the leaflets. The formation of the commissures may include folding each commissure tab of each leaflet into a series of overlapping layers such that each folded commissure tab is arranged outward of, in a lateral direction that is arranged tangent to a circumference of the frame of the prosthetic heart valve, a portion of the body of the leaflet that is directly connected to the commissure tab. The formation of the commissure may further include pairing each folded commissure tab of each leaflet with a folded commissure tab of another leaflet and securing at least a portion of the series of overlapping layers of each folded commissure tab in its folded configuration. Each commissure can then be attached to a respective commissure support portion of the frame of the prosthetic heart valve, either directly or via an attachment member configured to be coupled to the commissure support portion. The attaching can be performed with one or more fasteners such that the body of each leaflet is separated from each of the commissure support portion, the attachment member, and the one or more fasteners, by the folded commissure tab.

In this way, the folded commissure tabs are configured to offset the bending regions of the leaflets (e.g., bodies of the leaflets), away from the commissure support portions of the frame or attachment members attached thereto. As a result, the movable portions of the leaflets do not contact the commissure support portion or components attached thereto. Additionally, the fasteners (e.g., sutures) used to attach the commissure to the attachment member and/or to the commissure support portion extend through the tab portions and their folds, and do not penetrate the main bodies of the leaflets, including their movable or bendable regions. This further increases the stability and longevity of the commissure assembly (e.g., the leaflets experience reduced stress and the fasteners experience less wear).

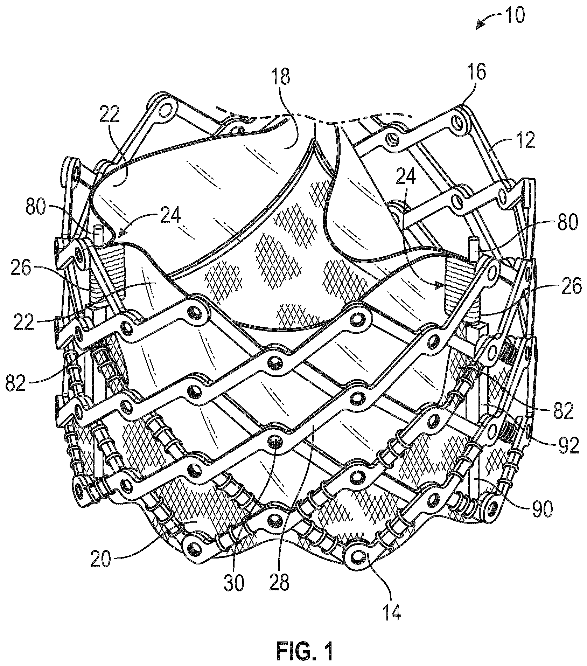

FIG. 1 shows an exemplary prosthetic heart valve 10, according to one embodiment. The prosthetic heart valve 10 can be radially compressible and expandable between a radially compressed configuration for delivery into a patient and a radially expanded configuration. In particular embodiments, the prosthetic heart valve 10 can be implanted within the native aortic annulus, although it also can be implanted at other locations in the heart, including within the native mitral valve, the native pulmonary valve, and the native tricuspid valve. The prosthetic heart valve 10 can include an annular stent or frame 12 having a first end 14 and a second end 16.

In the depicted embodiment, the first end 14 is an inflow end and the second end 16 is an outflow end. The outflow end 16 can be coupled to a delivery apparatus for delivering and implanting the prosthetic heart valve within the native aortic valve is a transfemoral, retrograde delivery approach. Thus, in the delivery configuration of the prosthetic heart valve, the outflow end 16 is the proximal-most end of the prosthetic valve. In other embodiments, the inflow end 14 can be coupled to the delivery apparatus, depending on the particular native valve being replaced and the delivery technique that is used (e.g., trans-septal, transapical, etc.). For example, the inflow end 14 can be coupled to the delivery apparatus (and therefore is the proximal-most end of the prosthetic heart valve in the delivery configuration) when delivering the prosthetic heart valve to the native mitral valve via a trans-septal delivery approach.

The frame 12 can be made of any of various suitable materials, such as stainless steel, a cobalt chromium alloy, or a nickel titanium alloy ("NiTi"), for example Nitinol. Referring again to FIG. 1, as shown, the frame 12 can include a plurality of interconnected struts 28 arranged in a lattice-type pattern. The struts 28 are shown as positioned diagonally, or offset at an angle relative to, and radially offset from, a longitudinal axis of the prosthetic heart valve 10 when the prosthetic heart valve 10 is in the expanded configuration. In other implementations, the struts 28 can be offset by a different amount than depicted in FIG. 1, or some or all of the struts 28 can be positioned parallel to the longitudinal axis of the prosthetic heart valve 10.

In the illustrated embodiment, the struts 28 are pivotably coupled to one another at one or more pivot joints along the length of each strut. For example, in the illustrated configuration, each of the struts 28 can be formed with apertures at opposing ends of the strut and apertures spaced along the length of the strut. Respective hinges can be formed at the locations where struts 28 overlap each other via fasteners or pivot members, such as rivets or pins 30 that extend through the apertures. The hinges can allow the struts 28 to pivot relative to one another as the frame 12 is radially expanded or compressed, such as during assembly, preparation, or implantation of the prosthetic heart valve 10.

In some embodiments, the frame 12 can be constructed by forming individual components (e.g., the struts and fasteners of the frame) and then mechanically assembling and connecting the individual components together. In other embodiments, the struts 28 are not coupled to each other with respective hinges but are otherwise pivotable or bendable relative to each other to permit radial expansion and contraction of the frame 12. For example, the frame 12 can be formed (e.g., via laser cutting, electroforming or physical vapor deposition) from a single piece of material (e.g., a metal tube). Further details regarding the construction of the frame and the prosthetic heart valve are described in U.S. Patent Application Publication Nos. 2018/0153689, 2018/0344456, and 2019/0060057, all of which are incorporated herein by reference.

The prosthetic heart valve 10 can also include a valvular structure 18 which is coupled to the frame 12 and configured to regulate the flow of blood through the prosthetic heart valve 10 from the inflow end 14 to the outflow end 16. The prosthetic heart valve 10 can further include a plurality of actuators 80 mounted to and equally spaced around the inner surface of the frame 12. The actuators are configured to apply expansion and compression to the frame for radially expanding and compressing the prosthetic valve.

In the illustrated embodiment, the actuators 80 are linear actuators, each of which comprises an inner member, or piston, 90 and an outer member, or cylinder, 92. The inner member 90 is pivotably coupled to a junction of the frame, such as at the first end 14, while the outer member 92 is pivotably coupled to another junction of the frame closer to the second end 16. Moving the inner member 90 proximally relative to the outer member 92 and/or moving the outer member 92 distally relative to the inner member 90 is effective to radially expand the prosthetic valve. Conversely, moving the inner member 90 distally relative to the outer member 92 and/or moving the outer member 92 proximally relative to the inner member 90 is effective to radially compress the prosthetic valve. The actuators 80 can include locking mechanisms that are configured to retain the prosthetic valve in an expanded state inside the patient's body.

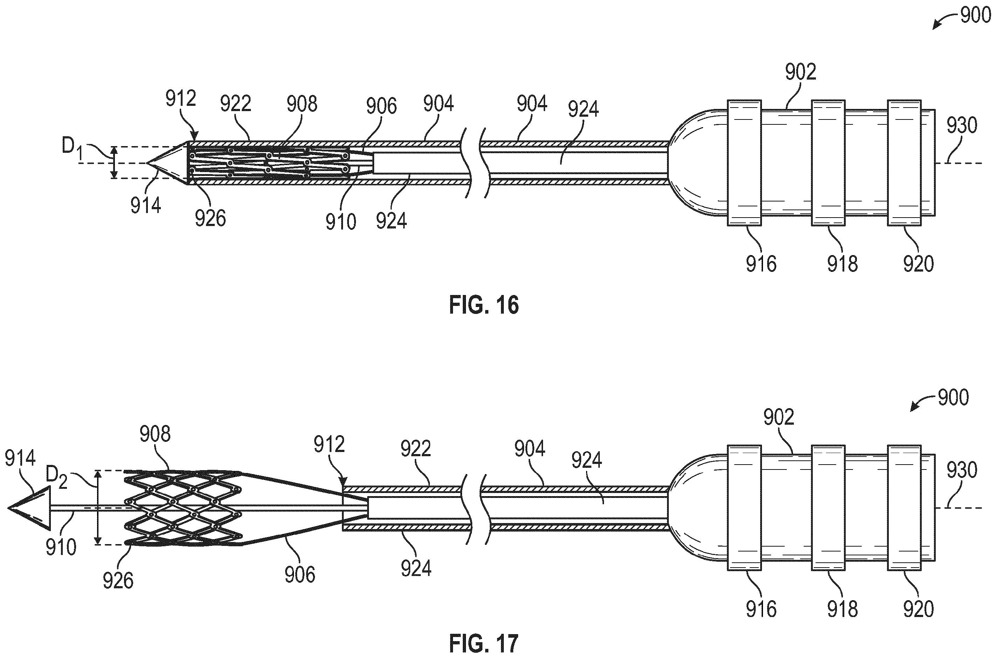

In some embodiments, each of the actuators 80 can be configured to form a releasable connection with one or more respective actuators of a delivery apparatus of a transcatheter delivery system (e.g., such as the delivery apparatus shown in FIGS. 16 and 17, as described below). The actuators of the delivery apparatus can transmit forces from a handle of the delivery apparatus to the actuators 80 for expanding or compressing the prosthetic valve. Further details of the actuators, locking mechanisms and delivery apparatuses for actuating the actuators can be found in U.S. Patent Application Publication Nos. 2018/0153689, 2019/0060057 and 2018/0325665, each of which is incorporated herein by reference in its entirety. Any of the actuators and locking mechanisms disclosed in the previously filed applications can be incorporated in any of the prosthetic valves disclosed herein. Further, any of the delivery apparatuses disclosed in the previously filed applications can be used to deliver and implant any of the prosthetic valves discloses herein.

In some embodiments, each of the actuators 80 can be used to support a respective commissure 24 (described below). As such, the actuators 80 can include commissure support portions for supporting and attaching commissures 24 of the valvular structure 18 to the frame 12, as described further herein.

The valvular structure 18 can include, for example, a leaflet assembly comprising one or more leaflets 22 (three leaflets 22 in the illustrated embodiment) made of a flexible material. The leaflets 22 of the leaflet assembly can be made from in whole or part, biological material, bio-compatible synthetic materials, or other such materials. Suitable biological material can include, for example, bovine pericardium (or pericardium from other sources). Each leaflet 22 includes two opposing commissure tabs arranged on opposite sides of a body of the leaflet. The body of the leaflet may be the portion of the leaflet that is adapted to bend and move during operation of the prosthetic heart valve 10. The commissure tabs of adjacent leaflets 22 can be arranged to form commissures 24, which can be, for example, mounted to commissure support portions of respective actuators 80. Further details regarding transcatheter prosthetic heart valves, including the manner in which the valvular structure can be coupled to the frame 12 of the prosthetic heart valve 10, can be found, for example, in U.S. Patent Nos. 6,730,118, 7,393,360, 7,510,575, 7,993,394, and 8,652,202, and U.S. Patent Application Publication No. 2018/0325665, all of which are incorporated herein by reference in their entireties.

In some embodiments, as shown in FIG. 1, the commissures 24 can be mounted (e.g., sutured) directly to commissure support portions of the actuators 80 of the frame 12 via commissure attachments 26. As one example, the commissure attachments 26 may include one or more stitches securing the commissures 24 to corresponding actuators 80. In other embodiments, the commissures 24 can be mounted to support struts or posts of the frame that are separate from the actuators 80. In still other embodiments, the commissures may be secured to an additional attachment member (as described further herein) and the attachment member is then secured to a commissure support portion of an actuator 80 or support struts or posts of the frame.

The prosthetic heart valve 10 can also include one or more skirts or sealing members. For example, as shown in FIG. 1, the prosthetic heart valve 10 can include an inner skirt 20 mounted on the inner surface of the frame 12. As shown in FIG. 1, the inner skirt 20 is a circumferential inner skirt that spans an entire circumference of the inner surface of the frame 12. The inner skirt 20 can function as a sealing member to prevent or decrease perivalvular leakage (e.g., when the valve is placed at the implantation site) and as an attachment surface to anchor the leaflets 22 to the frame 12. For example, the inflow (e.g., cusp) edges of the leaflets 22 can be sutured directly to the inner skirt 20, which in turn can be directly connected to selected struts 28 of the frame, such as with sutures, as shown in FIG. 1.

The prosthetic heart valve 10 can also include an outer skirt mounted on the outer surface of the frame 12 (not shown in FIG. 1). The outer skirt can function as a sealing member for the prosthetic valve by sealing against the tissue of the native valve annulus and helping to reduce paravalvular leakage past the prosthetic valve. The inner and outer skirts can be formed from any of various suitable biocompatible materials, including any of various synthetic materials (e.g., PET) or natural tissue (e.g., pericardial tissue). The inner and outer skirts can be mounted to the frame using sutures, an adhesive, welding, and/or other means for attaching the skirts to the frame.

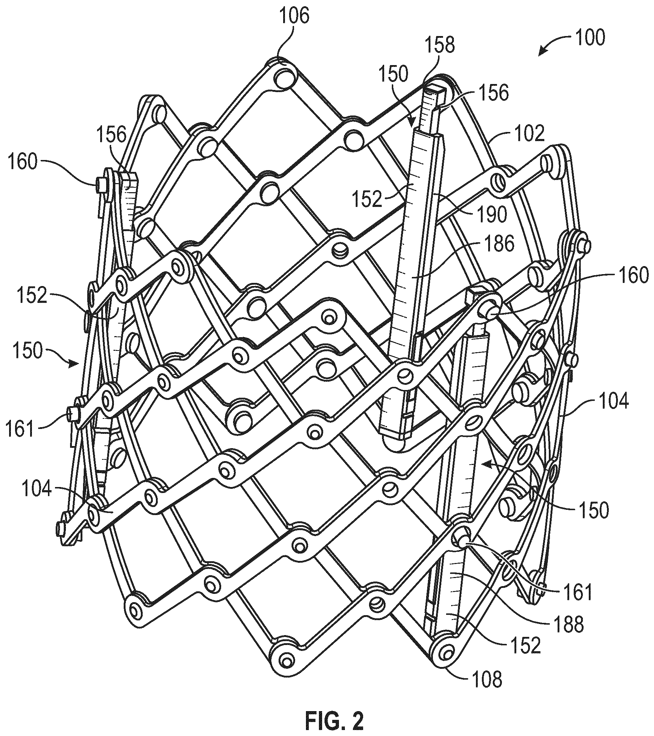

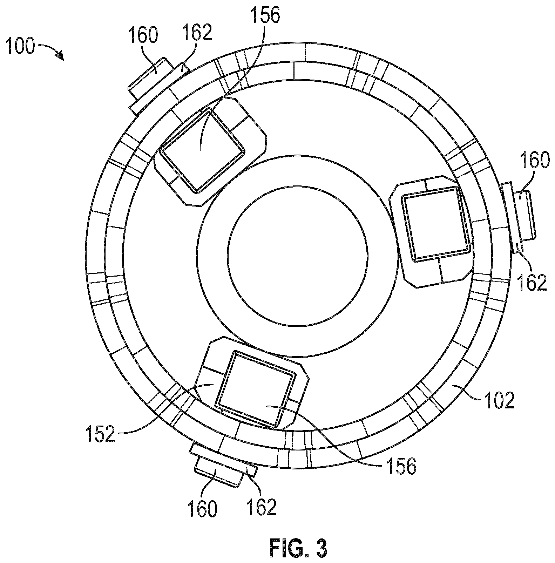

FIGS. 2 and 3 illustrate an exemplary embodiment of a prosthetic valve 100, according to another embodiment, the prosthetic valve 100 comprising a frame 102 and one or more expansion and locking mechanisms 150. The frame 102 comprises a plurality of pivotably connected struts 104 defining an inflow end 106 (which is the distal end of the frame in a delivery configuration for the illustrated embodiment) and an outflow end 108 (which is the proximal end of the frame in the delivery configuration for the illustrated embodiment). The struts 104 are pivotably connected to each other at a plurality of junctions that permit pivoting of the struts relative to each other when the frame 102 is radially compressed and expanded, as described above in connection with prosthetic valve 10.

The prosthetic valve 100 can include a valvular structure (e.g., valvular structure 18) and inner and/or outer skirts, as previously described, although these components are omitted from FIGS. 2 and 3 for purposes of illustration. The one or more expansion and locking mechanisms 150 can be used in lieu of or in addition to actuators 80 described above. The expansion and locking mechanisms 150 can be used to both radially expand and lock the frame 102 of prosthetic valve 100 in a radially expanded state. In some embodiments, the commissures of the leaflets may be attached to a commissure support portion of the expansion and locking mechanisms 150. In alternate embodiments, the commissures of the leaflets may be attached to additional commissure posts of the frame 102.

FIG. 2 shows three expansion and locking mechanisms 150 mounted to the frame 102 with the frame 102 shown in the radially expanded configuration. Though the illustrated embodiment shows three expansion and locking mechanisms 150 spaced apart from each other about the circumference of the frame, it should be noted that a prosthetic valve can comprise any number of expansion and locking mechanisms 150. For example, in some embodiments, a prosthetic valve can comprise a single expansion and locking mechanism, or two expansion and locking mechanisms, or four expansion and locking mechanisms, etc. The expansion and locking mechanisms 150 can be placed at any position about the circumference of the frame 102. For example, in some embodiments, such as the illustrated embodiment, the expansion and locking mechanisms 150 are equally spaced from one another about the circumference of the frame 502. In other embodiments, it can be advantageous to have two or more expansion and locking mechanisms situated adjacent to one another.

Each expansion and locking mechanism 150 can include an outer member in the form of a sleeve 152 having an inner lumen, cavity, or bore and an inner member 156 extending at least partially into the cavity. The sleeve 152 in the illustrated embodiment comprises an inner wall 186, an outer wall 188, and two side walls 190, each of which extends radially between a longitudinal edge of the inner wall 186 and an opposing longitudinal edge of the outer wall 188. The inner wall 186, the outer wall 188, and the two side walls 190 define the cavity, which is sized and shaped to receive the inner member 156.

The sleeve 152 in the illustrated embodiment has a rectangular shape in cross-section and the inner member 156 has a rectangular shape in cross-section corresponding to the shape of the bore. In other embodiments, the sleeve 152 and/or the inner member 156 can have a square cross-sectional profile. As shown in FIG. 3, the rectangular and/or square cross-sections can advantageously minimize the distance that the expansion and locking members extend into the lumen of the frame 102, which can reduce the overall crimp profile of the valve 100. However, in other embodiments, the sleeve and the inner member can have any of various corresponding shapes in cross-section, for example, circular, ovular, triangular, rectangular, square, or combinations thereof.

As best shown in FIG. 1, a distal end portion 158 of the inner member 156 can be coupled to the frame 102 at a first location via a fastener 160 that is affixed to and extends radially from the distal end portion 158 of the inner member 156. The fastener 160 can be for example, a rivet or pin. As shown, in some embodiments, the fastener 160 can extend through corresponding apertures at a junction of two overlapping struts 104 of the frame 102 and can serve as a pivot pin around which the two struts 104 can pivot relative to each other and the inner member 156. In some embodiments, an end cap or nut 162 (as shown in FIG. 3) can be disposed over an end portion of the fastener 160. The nut 162 can have a diameter greater than the diameter of the apertures to retain the fastener 160 within the apertures. In alternative embodiments, the inner member 156 need not comprise a fastener 160 and can be coupled to the frame 102 via other means of attachment such as welding, adhesives, etc.

The sleeve 152 can be coupled to the frame 102 at a second location, axially spaced from the first location. For example, in the illustrated embodiment, the inner member 156 is secured to the frame 102 near the distal or inflow end 106 of the frame and the sleeve 152 is secured to the frame 102 closer to or at the proximal or outflow end 108 of the frame, such as via a fastener 161 (e.g., a rivet or pint). The fastener 161 is affixed to and extends radially from the sleeve 152 through corresponding apertures at a junction of two overlapping struts 104 and can serve as a pivot pin around which the two struts 104 can pivot relative to each other and the sleeve 152. A nut 162 can be mounted on each fastener 161 to retain the fastener within the corresponding apertures. The expansion and locking mechanism 150 can be pivotably coupled to the frame 102 at any two axially spaced, circumferentially aligned locations on the frame.

The inner member 156 can be axially movable relative to the sleeve 152 in a proximal direction and in a distal direction, along a central longitudinal axis of the frame 102. As such, because the inner member 156 and the sleeve 152 are secured to the frame at axially spaced locations, moving the inner member 156 and the sleeve 152 axially with respect to one another in a telescoping manner can cause radial expansion or compression of the frame 102. For example, moving the inner member 156 proximally toward the outflow end 108 of the frame, while holding the sleeve 152 in a fixed position and/or moving the sleeve 152 distally toward the inflow end 106 of the frame can cause the frame 102 to foreshorten axially and expand radially. Conversely, moving the inner member 156 distally and/or moving the sleeve 152 proximally causes the frame 102 to elongate axially and compress radially.

A prosthetic valve 100 including one or more expansion and locking mechanisms 150 can be expanded in the following exemplary manner. Generally, the prosthetic valve 100 is placed in a radially compressed state and releasably coupled to a distal end portion of a delivery apparatus, and then advanced through the vasculature of a patient to a selected implantation site (e.g., the native aortic annulus). The prosthetic valve 100 can then be deployed at the implantation site and expanded and locked in the expanded configuration using the expansion and locking mechanisms 150. Further details regarding the prosthetic valve, the expansion and locking mechanisms, and delivery apparatuses for actuating the expansion and locking mechanism can be found in U.S. Provisional Application Nos. 62/928,291 and 62/950,005, the contents of which are incorporated herein by reference.

FIGS. 16 and 17 illustrate a delivery apparatus 900, according to one embodiment, adapted to deliver a prosthetic heart valve (e.g., prosthetic valve) 908, such as the prosthetic heart valve 10 illustrated in FIG. 1 and/or the prosthetic valve 100 illustrated in FIGS. 2-3, as described above, to a target implantation site in a patient. FIGS. 16-17 show the prosthetic valve 908 in different configurations relative to the delivery apparatus 900 during a valve implantation procedure. The prosthetic valve 908 can be releasably coupled to one or more components of the delivery apparatus 900, as described above and further below. It should be understood that the delivery apparatus 900 can be used to implant prosthetic devices other than prosthetic valves, such as stents or grafts.

The delivery apparatus 900 in the illustrated embodiment generally includes a handle 902, an elongate shaft 904 (which comprises an outer, or outermost, shaft in the illustrated embodiment) extending distally from the handle 902, an inner (e.g., innermost) shaft 910, an intermediate shaft 924 arranged coaxial with and between (in the radial direction which is perpendicular to a central longitudinal axis 930 of the delivery apparatus 900) the outer shaft 904 and the inner shaft 910, and at least one actuator assembly (e.g., member or actuator) 906 for expanding and compressing the prosthetic valve 908, the at least one actuator assembly 906 extending through the outer shaft 904 and distally outwardly from a distal end portion 912 of the outer shaft 904.

In some embodiments, the outer shaft 904, inner shaft 910, intermediate shaft 924, and/or actuator assembly 906 may make up a delivery apparatus catheter of the delivery apparatus 900, controlled by and attached to the handle 902.

The delivery apparatus 900 can include three actuator assemblies 906 (only two of the three are shown in FIGS. 16-17) releasably coupled to the prosthetic valve 908. However, in alternate embodiments, the delivery apparatus 900 may include more or less than three actuator assemblies 906 (e.g., one, two, four, or the like). As shown in FIGS. 16-17, the plurality of actuator assemblies 906 are circumferentially spaced apart from each other around a circumference of the delivery apparatus 900 and can extend axially through the outer shaft 904 from the handle 902 to the prosthetic valve 908.

In particular embodiments, each actuator assembly 906 can be releasably coupled to a corresponding actuator of the prosthetic valve (e.g., an actuator 80 as shown in FIG. 1 or a portion of an expansion and locking mechanism 150 as shown in FIGS. 2 and 3). Each actuator assembly 906 can include an inner member having a distal end releasably coupled to an inner member of an actuator of the prosthetic valve and an outer member having a distal end releasably coupled to an outer member of an actuator of the prosthetic valve. In another embodiment, each actuator assembly 906 can be an actuator assembly releasably coupled to the prosthetic valve 908 via a threaded sleeve.

In some embodiments, the intermediate shaft 924 may be adapted to house and organize the actuator assemblies 906. For example, the actuator assemblies 906 may be housed within and extend outwardly from a distal end of the intermediate shaft 924. In some embodiments, each actuator assembly 906 may be kept separate from the other actuator assemblies 906 within the intermediate shaft 924. For example, each actuator assembly 906 can extend through a separate lumen of the intermediate shaft 924.

As shown in FIGS. 16-17, a distal end of the inner shaft 910 may include a nosecone 914 which may be used to guide the delivery apparatus 900 through a lumen of a patient to a target implantation site for the prosthetic valve 908. The nosecone 914 may be arranged proximate to a distal end 926 of the prosthetic valve 908.

In use, the delivery apparatus 900 can be releasably coupled to the prosthetic valve 908 to produce radial expansion and compression of the frame of the prosthetic valve 908. In some embodiments, the actuator assemblies 906 of the delivery apparatus 900 can be configured to transfer pushing and/or pulling forces from the handle 902 of the delivery apparatus 900 to the prosthetic valve 908. For example, in some embodiments, the actuator assemblies 906 may have distal end portions that can be releasably connected to the prosthetic valve 908 via respective release-and-locking units.

In some embodiments, the outer shaft 904 of the delivery apparatus 900 can be configured as a steerable guide catheter having an adjustable curvature for use in steering the delivery apparatus 900 through the patient's vasculature. In particular embodiments, the outer shaft 904 can include a steerable distal section, the curvature of which can be adjusted by the operator to assist in guiding the apparatus through the patient's vasculature.

The outer shaft 904 and the actuator assemblies 906 can be moved relative to one another (axially and/or rotationally) to facilitate delivery and positioning of the prosthetic valve 908 at an implantation site in the patient's body.

In some embodiments, the distal end portion 912 of the outer shaft 904 can form and/or function as a sheath (e.g., capsule) 922 that is sized and shaped to receive and house the prosthetic valve 908 in a radially compressed state for delivery into and through a patient's vasculature. Once the prosthetic valve 908 is advanced to the implantation site or adjacent the implantation site, the prosthetic valve 908 can be advanced from the capsule 922 by retracting the outer shaft 904, and thus the capsule 922, axially, along central longitudinal axis 930, relative to the actuator assemblies 906 and the prosthetic valve 908. As such, the prosthetic valve 908 may be uncovered while the capsule 922 moves axially back towards the handle 902 (e.g., in a proximal direction along the central longitudinal axis 930). In alternative embodiments, the prosthetic valve 908 can be advanced from the capsule 922 by advancing the actuator assemblies 906 relative to the outer shaft 904, after which the prosthetic valve 908 can be radially expanded.

The advancement of the prosthetic valve 908 from the sheath by axially moving the actuator assemblies 906 relative to the outer shaft 904 or by retracting the outer shaft 904 relative to the actuator assemblies 906 may be actuated by operating a first knob 916 on the handle 902. The first knob 916 can be operatively connected to a proximal end portion of the outer shaft 904 and can be configured to retract the outer shaft 904 proximally relative to the actuator assemblies 906 to deploy the prosthetic valve 908 from the distal end portion 912 of the capsule 922 or operatively connected to proximal ends of the actuator assemblies 906 to advance the actuator assemblies 906 distally relative to the outer shaft 904 to deploy the prosthetic valve 908 from the distal end portion 912 of the capsule 922. The first knob 916 may be a slidable or rotatable adjustment element that is operatively connected to the actuator assemblies 906 and/or the outer shaft 904.

The handle 902 may include additional adjustment knobs, such as a second knob 918 and a third knob 920, as shown in FIGS. 16-17. In some embodiments, the second knob 918 may be operatively coupled to the actuator assemblies 906 and actuate the actuator assemblies 906 to adjust the prosthetic valve 908 from a non-expanded (or radially compressed) configuration (as shown in FIG. 16) to a radially expanded configuration, and vice versa.

In some embodiments, the third knob 920 may be operatively coupled to the actuator assemblies 906 and actuate the actuator assemblies 906 to disconnect from the prosthetic valve 908. As a result, the prosthetic valve 908 may be detached from the delivery apparatus 900 and implanted (e.g., deployed) at the target implantation site.

FIG. 16 shows the prosthetic valve 908 retained in a radially compressed state within the capsule 922 of the delivery apparatus 900. As such, in FIG. 16, the prosthetic valve 908 is in its radially compressed configuration having a smallest diameter, D1. The smallest diameter D1 may be approximately the same as an inner diameter of the capsule 922. The capsule 922 surrounding an outside of the prosthetic valve 908, as shown in FIG. 16, may maintain the prosthetic valve in the radially compressed configuration. As a result, the prosthetic valve 908 may be advanced through a patient's vasculature, for example, to the target implantation site via the delivery apparatus 900.

After reaching the target implantation site, the capsule 922 may be pulled (e.g., retracted) away from the nosecone 914 and the prosthetic valve 908, in a proximal direction along the central longitudinal axis 930 of the delivery apparatus 900, to uncover the prosthetic valve 908. In alternate embodiments, the actuator assemblies 906 may be advanced, in the distal direction, to move the prosthetic valve 908 out of the capsule 922.

FIG. 17 shows the prosthetic valve 308 in the uncovered (e.g., unsheathed) state where it is arranged outside of the capsule 922. At this state, the prosthetic valve 908 is not actively expanded via the actuator assemblies 906. However, since it is no longer bound by (e.g., retained within) the capsule 922, the prosthetic valve 908 may assume a partially expanded diameter D2 which is larger than the smallest diameter D1 due to the inherent resiliency of the struts of the frame. It should be noted that the extent of expansion of the prosthetic valve 908, from the compressed, smallest diameter D1 (FIG. 16) to the partially expanded diameter D2 (FIG. 17) may be exaggerated in FIG. 17 for the purposes of illustration. After being deployed from the capsule 922, as shown in FIG. 17, the prosthetic valve 908 can then be radially expanded and implanted, by actuation of the actuator assemblies 906.

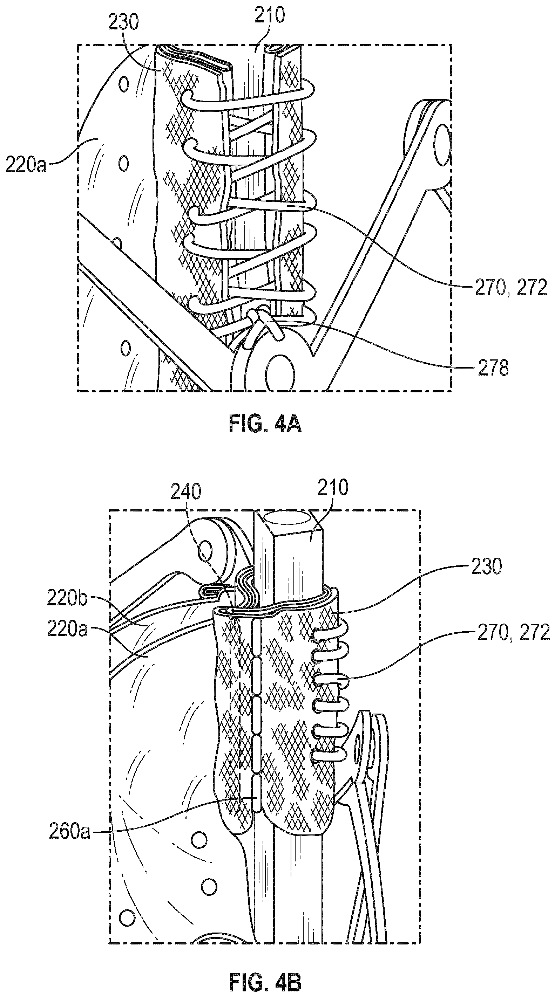

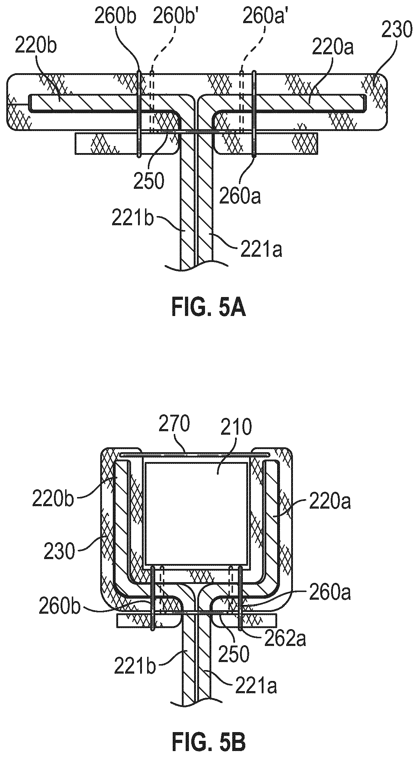

Turning now to FIGS. 4A-5B, example commissure tab assemblies and attachments of commissure tab assemblies to a commissure support portion of a frame of a prosthetic valve, such as a commissure post or other support structure of the frame of the prosthetic valve, are shown. FIGS. 4A and 4B show an example commissure tab assembly from two different view angles and FIGS. 5A and 5B show example cross-sectional views of the commissure tab assembly in two different states (e.g., prior to wrapping the commissure around a support structure of the frame and after wrapping the commissure at least partially around a support structure of the frame, respectively).

A commissure tab assembly may be pre-assembled prior to attachment thereof to the frame by performing a pre-assembly process. The pre-assembly process includes, in one example, forming a primary suture line 250 having a plurality of in-and-out stitches extending through, in order (or in the reverse order, e.g., starting from the last-listed element and extending through the following list of elements, in reverse, to the first-listed element), a first portion on a first side of a support strip (e.g., a flexible cloth/fabric) 230, a first commissure tab 220a, a second commissure tab 220b (where commissure tabs 220a and 220b are two commissure tabs of adjacent leaflets, e.g., leaflets 221a and 221b), and a second portion on a second side of the support strip 230. As used herein, the term "suture line" can also be referred to as a "stitch line." Optionally, a reinforcement member 240 (one of which is shown in FIG. 4B) can be positioned between each commissure tab 220a, 220b and an adjacent portion of the support strip 230 and can be stitched to the commissure tabs with the primary suture line. The second side of the support is opposite the first side relative to a center of the support strip (e.g., a centerline that divides a width/longest dimension of the strip in half).

The support strip 230 may be a strip of any suitable material (e.g., fabric), which may include material that is stronger (e.g., more resilient to tearing and/or deforming) than a material used for forming the leaflets and/or the commissure tab portions of the leaflets of the prosthetic valve. In one example, the support strip 230 is a polyethylene terephthalate (PET) fabric, although various other suitable biocompatible fabrics can be used.

Returning to the formation of the pre-assembled commissure, additional, secondary suture lines 260a and 260b having a plurality of in-and-out stitches may be extended, each, in order (or in the reverse order): through respective first layers of the support strip 230, optionally through respective reinforcing members 240 (when included in the commissure), through respective second layers of the support strip 230, through respective commissure tabs 220a and 220b and through respective third layers of the support strip 230 in a radial direction.

Secondary suture lines 260a' and 260b' in FIGS. 5A and 5B show example alternative placements for the secondary suture lines 260a and 260b, respectively. The alternative secondary suture lines 260a' and 260b' pass through only two of the above-described layers of the support strip. In still other examples, secondary suture lines may be positioned in both locations (e.g., a combination of four or three of the suture lines 260a, 260b, 260a', and 260b' may be used) in order to provide additional reinforcement of the coupling of the support strip to itself and the commissure tabs 220a and 220b.

The pre-assembled commissure tab assembly, assembled as described in any of the examples above, may be attached to a corresponding commissure post 210 (or other commissure support structure, such as a commissure support portion of an actuator, post, or expansion and locking mechanism) of the frame. The commissure post 210 can be a component of an actuator 80 of the prosthetic valve 10. For example, the upper portion of outer member 92 (FIG. 1) can serve as the commissure post 210 (e.g., commissure support portion). In alternative embodiments, the prosthetic valve 10 can include commissure posts separate from the actuators. The separate commissure posts can be mounted to the inner surface of the frame 12, or can be integral portions of the frame, at locations circumferentially spaced from the actuators.

The commissure tab assembly may be attached to the commissure post 210 by forming a tertiary suture line 270 along the support strip 230 (e.g., each of two opposing sides of the support strip) adjacent two circumferentially opposite sides of the commissure post 210, for example by forming shoelace stitches around the post 210 and knotting both ends of the suture 272 (e.g., forming a knot 278 at the top and/or bottom end). The stitches formed by tertiary suture line 270 extend between opposing sides of the support strip across a first side of the post 210, and may optionally extend at least 360 degrees around the post 210.

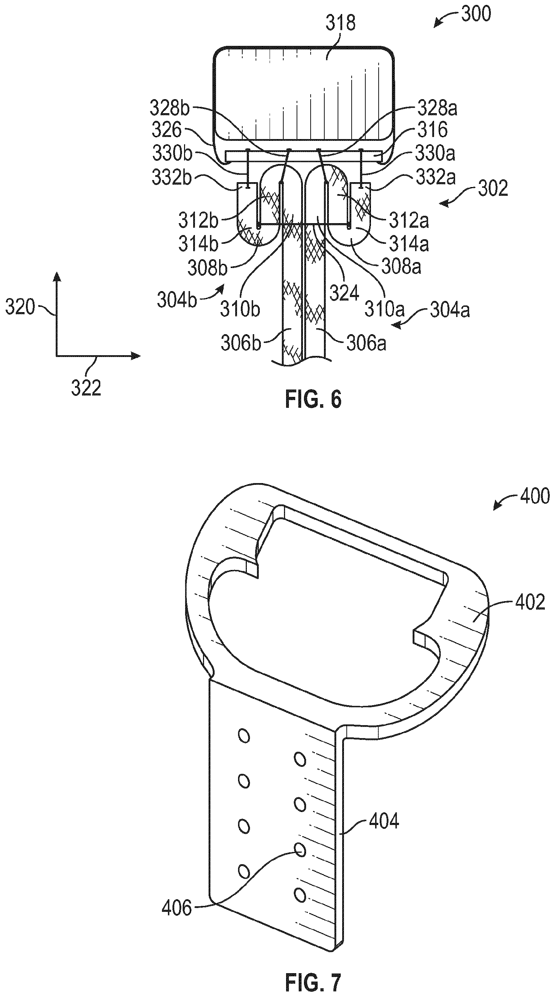

FIGS. 6-14 show example embodiments of a commissure assembly (e.g., commissure attached to a commissure support portion) including a pair of folded commissure tabs that are secured directly to a commissure support portion of a frame of a prosthetic heart valve or to an attachment member (also referred to as a commissure support element or member) that is then secured to the commissure support portion. For example, in some embodiments, each commissure tab of each leaflet is folded into a series of overlapping layers, paired with a similarly folded commissure tab of another leaflet, and secured to retain the commissure tab in its folded configuration. The pair of folded commissure tabs, forming a commissure, is then attached to a corresponding support portion of a frame of the prosthetic heart valve, either directly or via an attachment member configured to be coupled to the commissure support portion.

Though FIGS. 6, 8-10, and 12-14 show one commissure secured to one commissure support portion of a frame of a prosthetic heart valve, a prosthetic heart valve can include additional commissures assembled in the same manner as shown in these figures. For example, for a prosthetic heart valve including a leaflet assembly with three leaflets, there can be three commissures, each secured to a respective commissure support portion of the frame of the prosthetic heart valve.

In some embodiments, as shown in FIGS. 6, 8-10, and 12-14, the pair of folded commissure tabs are folded such that the folded commissure tabs are positioned between and separate the main bodies of the leaflets connected to the commissure tabs from the attachment member and/or commissure support portion of the frame. For example, each folded commissure tab can be arranged outward of, in a lateral direction arranged tangent to a circumference of the prosthetic heart valve, a portion of the body of the leaflet that is directly connected to the commissure tab. Further, each folded commissure tab can be arranged between, in a radial direction relative to a central longitudinal axis of the prosthetic heart valve, the main bodies of the leaflets and the attachment member and/or commissure support portion of the frame.

Further, the pair of folded commissure tabs can be folded so that various fasteners (e.g., sutures) used to secure the pair of folded commissure tabs to one another and to the attachment member and/or commissure support portion are arranged within the folds and do not contact the main bodies of the leaflets, even during operation of the prosthetic valve (e.g., during diastolic and systolic cycles during operation of the prosthetic valve). In this way, a separation is created between moving portions of the leaflets (e.g., the main bodies that move during operation of the prosthetic valve) and the fasteners (e.g., sutures). As a result, wear to the fasteners during use is reduced and a load carried by the fasteners is reduced, thereby increasing the stability and longevity of the entire leaflet assembly of the prosthetic valve.

Turning first to FIGS. 6-8, a first embodiment of a commissure assembly 300 for a prosthetic heart valve is shown. The prosthetic heart valve can be one of the prosthetic heart valves 10 (FIG. 1) or 100 (FIGS. 2-3) disclosed herein. The commissure assembly 300 comprises a commissure 302 formed from commissure tabs of two adjacent leaflets. Each leaflet includes two opposing commissure tabs arranged on opposite sides of a main body of the leaflet (only one commissure tab on one side of the main body of each leaflet of the commissure 302 is shown in FIG. 6). For example, as shown in FIG. 6, a first leaflet 304a includes a first main body (e.g., body) 306a and a first commissure tab 308a arranged on (and directly connected to and continuous with) a first side of the first main body 306a and a second leaflet 304b includes a second main body 306b and a second commissure tab 308b arranged on a first side of the second main body 306b. The main bodies 306a and 306b are configured to move during operation of the prosthetic heart valve (e.g., when implanted in a heart of a patient). For example, the portions of the main bodies 306a and 306b shown in FIG. 6 may move away from and toward one another during systolic and diastolic cycles, respectively, of the heart.

As shown in FIG. 6, the first commissure tab 308a comprises a first portion 310a that is directly connected to and extends continuously and radially outward (relative to a radial direction 320 that is relative to a central longitudinal axis of the prosthetic heart valve) from the first main body 306a. The first portion 310a can further extend toward an attachment member 316 and/or commissure support portion 318 of a frame of the prosthetic heart valve to which the first commissure tab 308a is coupled, as explained further below. The first commissure tab 308a is then folded over itself (folded over the first portion 310a) to form a second portion 312a. The second portion 312a that is folded over the first portion 312a extends radially inward toward the first main body 306a. The first commissure tab 308a is then folded over itself (folded over the second portion 312a) again to form a third portion 314a. The third portion 314a extends radially outward toward the attachment member 316 and/or commissure support portion 318. A majority of each of (e.g., portions between the bends connecting the portions) the first portion 310a, second portion 312a, and third portion 314a are arranged in parallel with one another and overlap in a lateral direction 322 which is arranged tangent to a circumference of the frame of the prosthetic heart valve.

Similarly, the second commissure tab 308b comprises a first portion 310b that is directly connected to the second main body 306b and extends radially outward from the second main body 306b and toward the attachment member 316 and commissure support portion 318 to which the folded second commissure tab 308b is coupled, a second portion 312b that is folded over the first portion 310b and extends radially inward and toward the second main body 306b, and a third portion 314b that is folded over the second portion 312b and extends radially outward and toward the attachment member 316 and commissure support portion 318. As shown in FIG. 6, a majority of each of the first portion 310b, second portion 312b, and third portion 314b are arranged in parallel with one another and overlap in the lateral direction 322.

A first fastener 324 is wrapped around the second portion 312a, the first portion 310a, the first portion 310b, and the second portion 312b to secure the first commissure tab 308a and the second commissure tab 308b to one another, forming the commissure 302. The first fastener 324 may be tightened so that all the folded portions that it wraps around are touching and held against one another. Thus, in FIG. 6, the folded portions arranged within and held together by the first fastener 324 are shown separated from one another for illustration purposes only and may actually be pressed against one another without gaps arranged therebetween. In some embodiments, the first fastener 324 can be a band that is wrapped around the indicated portions of the first commissure tab 308a and the second commissure tab 308b. For example, in some embodiments, the band may comprise a suture material, cloth material, polymer, metal, and/or wire. Thus, the band may be rigid or flexible. In some embodiments the band may be an injected or stamped metal or polymeric part. In other embodiments, the first fastener 324 can be a suture.

The first fastener 324 may create a bending point for the working portions of the leaflets 304a and 304b (e.g., main bodies 306a and 306b). As such, the main bodies 306a and 306b, arranged radially inward (relative to the radial direction 320 and the central longitudinal axis of the prosthetic heart valve) of the first fastener 324, can bend and move during operation while the portions of the folded commissure tabs 308a and 308b that are arranged radially outward of the first fastener 324 remain stationary (e.g., no movement). For example, the main bodies 306a and 306b of the leaflets, when moving between open and closed positions during the systolic and diastolic phases, bend around a region which is proximate to the inner folds (e.g., bends) formed between the second portions 312a and 312b and third portions 314a and 314b, respectively, at a position which is radially inward of the first fastener 324.

As shown in FIG. 6, the stationary, folded commissure tabs 308a and 308b are positioned between, in the radial direction 320, the main bodies 306a and 306b and the attachment member 316 (and commissure support portion 318), thereby separating the bending regions of the leaflets 304a and 304b (which are the load carrying regions of the leaflets) from the attachment member 316 and commissure support portion 318 of the frame. As a result, the stability and longevity of the leaflet assembly of the prosthetic heart valve is increased, as discussed further below.

The resulting six-layered commissure 302 can be pre-assembled and then coupled to the commissure support portion 318 of the frame or to the attachment member 316 that is attached (or configured to be attached) to the commissure support portion 318. In some embodiments, the commissure support portion 318 can be a portion of an actuator of the frame, such as actuator 80 shown in FIG. 1 or expansion and locking mechanism 150 shown in FIGS. 2-3.

According to some embodiments, the attachment member 316 is a fabric strip that is positioned over an inner side of the commissure support portion 318 and attached thereto, for example via one or more second fasteners 326. In some embodiments the one or more second fasteners 326 may be sutures (e.g., post sutures) that connect to the attachment member 316 and wrap around an outer side of the commissure support portion 318.

According to other embodiments, the attachment member 316 can be a rigid plate (e.g., base plate) or wire-form body configured to couple to the commissure support portion 318.

FIG. 7 shows an embodiment of rigid plate-type attachment member 400, which can be used as the attachment member 316 in FIG. 6 in some embodiments. The attachment member 400 can also be used as the attachment member in the other commissure assembly embodiments disclosed herein.

As shown in FIG. 7, the attachment member 400 comprises an upper collar 402 that is configured to engage with a commissure support portion of a frame (e.g., commissure support portion 318 shown in FIG. 6). The attachment member 400 further comprises a plate 404 that is configured to extend substantially in parallel to, and radially offset from, the commissure support portion. As shown in FIG. 7, the plate 404 can be provided with a plurality of apertures 406 that can be utilized to attach the attachment member 400 to the commissure support portion of the frame, and/or to attach a commissure (e.g., commissure 302) to the attachment member 400. Further details on a rigid plate-type attachment member may be found in International Application No. PCT/US19/61392, the contents of which are incorporated by reference herein.

Returning to FIG. 6, the commissure 302 can be secured to the attachment member 316 (as shown in FIG. 6) or directly to the commissure support portion 318 of the frame via one or more additional fasteners. As shown in FIG. 6, in some embodiments, third fasteners 328a and 328b (e.g., sutures) can extend between an interior fold of each folded commissure tab 308a and 308b and the attachment member 316 to secure the commissure 302 to the attachment member 316. For example, the third fastener 328a can extend from an inner edge of a bend (e.g., fold) between the first portion 310a and second portion 312a of the first folded commissure tab 308a, through a thickness of the bend, and to the attachment member 316 and the third fastener 328b can extend from an inner edge of a bend (e.g., fold) between the first portion 310b and second portion 312b of the second folded commissure tab 308b, through a thickness of the bend, and to the attachment member 316.

In some embodiments, when the attachment member 316 is the attachment member 400 of FIG. 7, the third fasteners 328a and 328b may extend through corresponding apertures 406 in the attachment member to secure the commissure 302 to the attachment member 400. In some embodiments, the attachment member 316 and the attachment member 400 can be used together. For example, the attachment member 316 can be disposed between the plate 404 and the folds of the leaflets. Then, the third fasteners 328a, 328b can extend through the leaflets, the attachment member 316 and respective openings 406 of the plate 404.

In some embodiments, additional, fourth fasteners 330a and 330b (e.g., sutures) can be utilized to secure free ends 332a and 332b of each of the commissure tabs 308a and 308b, respectively, to the attachment member 316 or the second fastener 326 (secured around the commissure support portion 318), in order to retain the third portions 314a and 314b folded over the respective second portions 312a and 312b of the commissure tabs 308a and 308b. The free ends 332a and 332b can be referred to as outermost ends of the respective commissure tabs 308a and 308b, the outermost ends arranged furthest away from respective main bodies 306a and 306b, along a length of the commissure tabs 308a and 308b.

In particular embodiments, the pre-assembled commissure 302 can include the third fasteners 328a, 328b and the fourth fasteners 330a, 330b connecting the leaflets to the attachment member 316 and/or attachment member 400. The pre-assembled commissure 302 can then be connected to the commissure support member 318, such as using fasteners 326 (e.g., if the attachment member 316 is a fabric or similar material), or by placing the collar 402 over the commissure support member 318 (if an attachment member 400 is used).

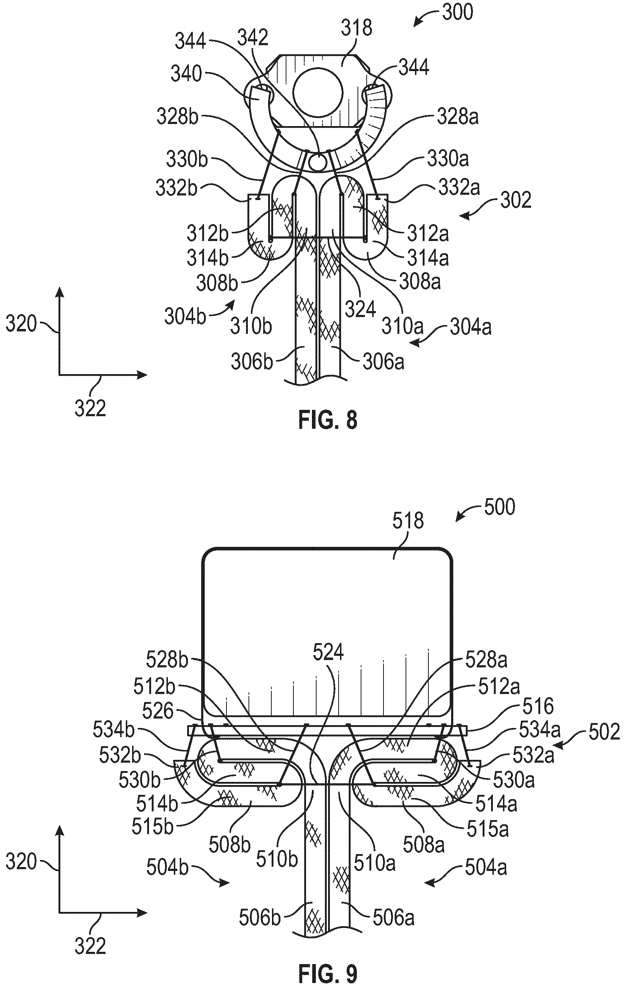

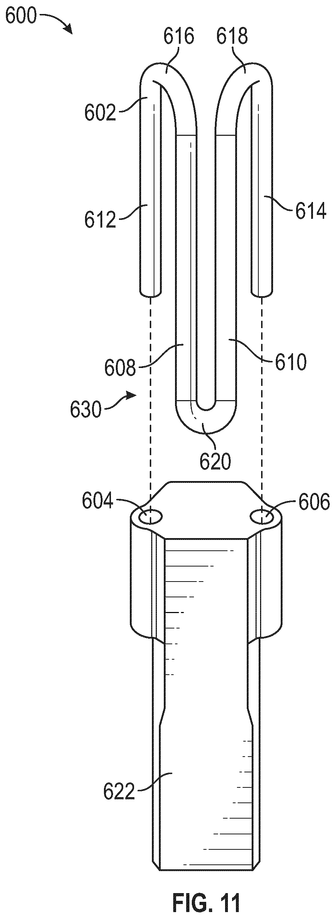

FIG. 8 shows the first embodiment of the commissure 302 shown in FIG. 6, attached to an alternate attachment member. In FIG. 8, the commissure 302 is secured to a wire-form attachment member 340 comprising a wire-form body. Another exemplary embodiment of a wire-form attachment member is shown in FIG. 11, as described further below. The wire-form attachment member 340 may be similar to the wire-form shown in FIG. 11, but may include a single, central axial arm 342 (alternative wire-forms or laser-cut or tube-cut members with alternate shapes are also possible). The commissure 302 can then be coupled to the wire-form attachment member 340 in a similar manner to that shown in FIG. 6, as described above. For example, one or more of the fasteners discussed above may be wrapped around and secured to a portion of the wire-form body of the wire-form attachment member 340. The wire-form member 340 may include coupling arms (not visible in FIG. 8) configured to fit within corresponding channels 344 of the commissure support portion 318, similar to as described below with reference to FIG. 11.

In this way, the folded commissure tabs 308a and 308b of the first embodiment shown in FIGS. 6 and 8 are configured to offset the bending regions of the leaflets 304a and 304b (e.g., main bodies 306a and 306b), which are the load carrying regions of the leaflets, away from the commissure support portions 318 of the frame or attachment members attached thereto (e.g., attachment member 316 or attachment member 340). As a result, the movable portions of the leaflets do not contact the commissure support portion 318 or components attached thereto. Further, the folded commissure tab portions together form a "virtual window", having the leaflets and their movable or bendable regions, positioned therein. For example, each folded commissure tab is arranged outward of, in a lateral direction arranged tangent to a circumference of the prosthetic heart valve, a portion of the main body of the leaflet that is directly connected to the commissure tab. Thus, during operation of the prosthetic heart valve, the movable portions of the leaflets are maintained interior to the folded commissure tabs (in the lateral direction) and do not come into contact with the commissure support portion of the frame or the attachment member.

Additionally, the fasteners (e.g., sutures) used to attach the commissure 302 to the attachment member and/or commissure support portion extend through the tab portions and their folds, and do not penetrate the main bodies of the leaflets, including their movable or bendable regions. This further increases the stability and longevity of the commissure assembly (e.g., the leaflets experience reduced stress and the fasteners experience less wear).

FIGS. 9, 10, and 12 show a second embodiment of a commissure assembly 500 for a prosthetic heart valve. The prosthetic heart valve can be one of the prosthetic heart valves 10 (FIG. 1) or 100 (FIGS. 2-3) disclosed herein. The commissure assembly 500 comprises a commissure 502 formed from commissure tabs of two adjacent leaflets. As explained above with reference to FIGS. 6 and 8, each leaflet includes two opposing commissure tabs arranged on opposite sides of a main body of the leaflet (only one commissure tab on one side of the main body of each leaflet of the commissure 502 is shown in FIGS. 9, 10, and 12).

For example, as shown in FIG. 9, a first leaflet 504a includes a first main body (e.g., body) 506a and a first commissure tab 508a arranged on (and continuous with and directly connected to) a first side of the first main body 506a and a second leaflet 504b includes a second main body 506b and a second commissure tab 508b arranged on a first side of the second main body 506b. The main bodies 506a and 506b are configured to move during operation of the prosthetic heart valve (e.g., when implanted in a heart of a patient), as explained above.

As shown in FIG. 9, the first commissure tab 508a comprises a first portion 510a that is directly connected to and extends continuously and radially outward (relative to the radial direction 320) from the first main body 506a. The first portion 510a can further extend toward an attachment member 516 (which may be the same or similar to the attachment member 316 described above with reference to FIGS. 6 and 8) and/or commissure support portion 518 of a frame of the prosthetic heart valve to which the first commissure tab 508a is coupled (which may be the same or similar to the commissure support portion 318 of FIGS. 6 and 8, as described above). The first commissure tab 508a is then folded over from the first portion 510a (e.g., at, approximately, a 90 degree angle) to extend in the lateral direction 322, away from the first portion 510a, to form a second portion 512a. As shown in FIG. 6, the second portion 512a extends substantially parallel to an inner surface of the commissure support portion 518 of the frame when attached (either directly or indirectly) thereto.

The first commissure tab 508a is then folded over itself (folded over the second portion 512a) to form a third portion 514a. The third portion 514a extends laterally inward, toward the first portion 510a. The first commissure tab 508a is yet again folded over itself (folded over the third portion 514a) to form a fourth portion 515a. The fourth portion 515a extends laterally outward, asway from the first portion 510a. A majority of each of (e.g., portions between the bends or folds connecting the portions) the second portion 512a, third portion 514a, and fourth portion 515a are arranged in parallel with one another and overlap in the radial direction 320. Further, the overlapping layers, including the second portion 512a, third portion 514a, and fourth portion 515a, all wind consecutively and are stacked in a consecutive fashion in the radial direction.

Similarly, the second commissure tab 508b comprises a first portion 510b that is directly connected to the second main body 506b of the second leaflet 504b and extends radially outward from the second main body 506b and toward the attachment member 516 and commissure support portion 518 to which the folded second commissure tab 508b is coupled, a second portion 512b that is folded away from the first portion 510b and extends laterally outward, in the lateral direction 322, from the first portion 510b, a third portion 514b that is folded over the second portion 512b and extends laterally inward toward the first portion 510b, and a fourth portion 515b that is folded over the third portion 514b and extends laterally outward and away from the first portion 510b. A majority of each of the second portion 512b, third portion 514b, and fourth portion 515b are arranged in parallel with one another and overlap in the radial direction 320.

As shown in FIG. 9, free ends 532a and 532b of each of the fourth portions 515a and 515b can further extend radially outward, toward the commissure support portion 518 of the frame when mounted thereto, and over a fold (e.g., bend) formed between the respective second portions 512a and 512b and third portions 514a and 514b. The free ends 532a and 532b can each be arranged exterior to folds of the corresponding folded commissure tab.

A first fastener 524 can be wrapped around an inner edge of a first bend (e.g., fold) between the third portion 514a and fourth portion 515a of the first commissure tab 508a, the first portion 510a, the first portion 510b, and an inner edge of a second bend (e.g., fold) between the third portion 514b and the fourth portion 515b of the second commissure tab 508b, thereby forming the commissure 502. The first fastener 524 may be tightened so that all the folded portions that it wraps around are touching and held against one another. In some embodiments, the first fastener 324 can be a band, as described above. In other embodiments, the first fastener 324 can be a suture.

Similar to the first fastener 324 of FIGS. 6 and 8, the first fastener 524 can create a bending point for the working portions of the leaflets 504a and 504b (e.g., main bodies 506a and 506b). As such, the main bodies 506a and 506b, arranged radially inward (relative to the radial direction and the central longitudinal axis of the prosthetic heart valve) of the first fastener 524, can bend and move during operation while the portions of the folded commissure tabs 508a and 508b that are arranged radially outward of the first fastener 524 remain stationary (e.g., no movement). For example, the main bodies 506a and 506b of the leaflets, when moving between open and closed positions during the systolic and diastolic phases, bend around a region which is proximate to the outer folds formed between the third portions 514a and 514b and fourth portions 515a and 515b, respectively, at a position which is radially inward of the first fastener 324.

Similar to the embodiment of FIGS. 6 and 8, the stationary, folded commissure tabs 508a and 508b are positioned between, in the radial direction 320, the main bodies 506a and 506b and the attachment member 516 (and commissure support portion 518), thereby separating the bending regions of the leaflets 504a and 504b (which are the load carrying regions of the leaflets) from the attachment member 516 and commissure support portion 518 of the frame. As a result, the stability and longevity of the leaflet assembly of the prosthetic heart valve is increased.

The resulting commissure 502 can be pre-assembled and then coupled to the commissure support portion 518 of the frame or to the attachment member 516 that is attached (or configured to be attached) to the commissure support portion 518. In some embodiments, the commissure support portion 518 can be a portion of an actuator of the frame, such as actuator 80 shown in FIG. 1 or expansion and locking mechanism 150 shown in FIGS. 2-3.

According to some embodiments, the commissure 502 can be pre-assembled such that the folded portions of the commissure tabs 508a and 508b extend in the same direction as the main bodies 506a and 506b of the leaflets 504a and 504b (e.g., in the radial direction 320) when positioned in a prosthetic heart valve, but prior to attachment to the frame. Then, during the attachment (e.g., securing) of the commissure 502 to the commissure support portion 518 of the frame (either directly or indirectly through the attachment member 516), the folded portions of the commissure tabs 508a and 508b can transition to being oriented in the lateral direction 322, substantially parallel to the inner surface of the commissure support portion 518 of the frame (as shown in FIGS. 9, 10, and 12).

According to some embodiments, the attachment member 516 is a fabric strip that is positioned over an inner side of the commissure support portion 518 and attached thereto, for example via one or more fasteners 526. In some embodiments the one or more fasteners 526 may be sutures (e.g., post sutures) that connect to the attachment member 516 and wrap around an outer side of the commissure support portion 518.

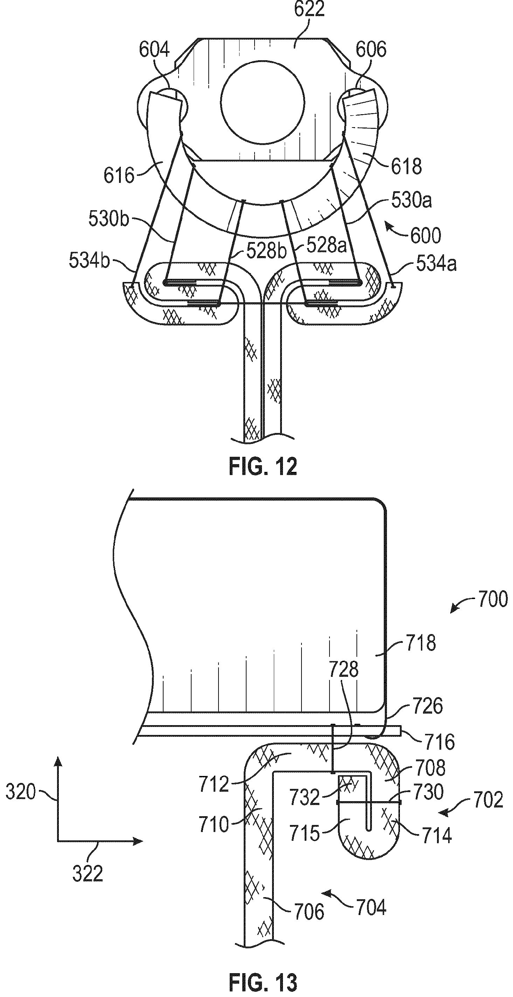

According to other embodiments, the attachment member 516 is a rigid plate (such as rigid plate-type attachment member 400 shown in FIG. 7) or a wire-form attachment member configured to couple to the commissure support portion 518 (such as the wire-form attachment member 340 shown in FIG. 8 or the wire-form attachment member 600 shown in FIGS. 11 and 12, as described further below). In some embodiments, the commissure can include a fabric attachment member and a more rigid attachment member, such as a plate type attachment 400 or a wire-form attachment member.

In some embodiments, the commissure assembly 500 includes second fasteners 528a and 528b, third fasteners 530a and 530b, and fourth fasteners 534a and 534b to secure the commissure 502 to the attachment member 516 (as shown in FIG. 9) or directly to the commissure support portion 518 of the frame. In other embodiments, the fourth fasteners 534a and 534b may be omitted. In some embodiments, the second, third, and/or fourth fasteners can be sutures.

Each of the second fasteners 528a and 528b can extend through two overlapping portions (second portions 512a and 512b and third portions 514a and 514b, respectively) and the attachment member 516. For example, each of the second fasteners 528a and 528b can extend from an inner edge of a bend (e.g., fold) between a respective third portion 514a and 514b and respective fourth portion 515a and 515b, through a thickness of each of the respective third portion 514a and 514b and second portion 512a and 512b, and to the attachment member 516.

Each of the third fasteners 530a and 530b can extend from an inner edge of a bend (e.g., fold) between a respective second portion 512a and 512b and third portion 514a and 514b, through a thickness of the respective second portion 512a and 512b, and to the attachment member 516.

In some embodiments, the fourth fasteners 534a and 534b can be utilized to secure a respective free end 532a and 532b of the fourth portions 515a and 515b, respectively, to the attachment member 516, in order to retain the fourth portions 515a and 515b extended radially outward over the fold between the respective second portions 512a and 512b and third portions 514a and 514b.

The second fasteners 528a and 528b may be the main, load bearing fasteners of the commissure 502 (e.g., hold a majority of the load of the leaflets). Since the second fasteners 528a and 528b extend through two overlapping portions of the commissure tabs (as compared to only one, as shown in the embodiment of FIGS. 6 and 8), these fasteners may be more robust and less likely to wear over time. As shown in FIG. 9, the outer, free ends of the attachment member 516 are positioned away from the main, load bearing second fasteners 528a and 528b. This may further increase the robustness of the second fasteners 528a and 528b.

In some embodiments, the second fasteners 528a and 528b, third fasteners 530a and 530b, and/or fourth fasteners 534a and 534b may extend through and secure to corresponding apertures in the attachment member 516 to secure the commissure 502 to the attachment member 516, for example when the attachment member 516 is a plate-type attachment member having one or more apertures arranged therein (such as attachment member 400 shown in FIG. 7).

In particular embodiments, the pre-assembled commissure 502 can include the second fasteners 328a, 328b, the third fasteners 530a, 530b, and the fourth fasteners 534a, 534b connecting the leaflets to the attachment member 516 and/or attachment member 400. The pre-assembled commissure 502 can then be connected to the commissure support member 518, such as using fasteners 526 (e.g., if the attachment member 516 is a fabric or similar material), or by placing the collar 402 over the commissure support member 518 (if an attachment member 400 is used).

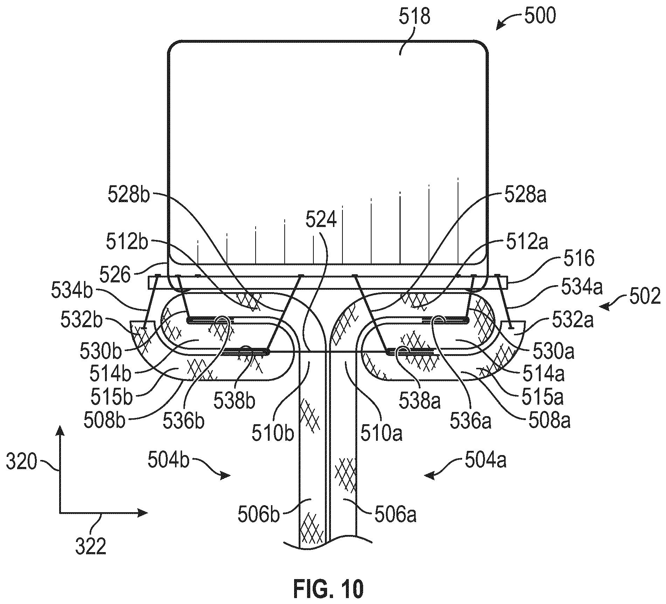

FIG. 10 shows another embodiment of the commissure assembly 500, which can be identical to the commissure assembly 500 described above with reference to FIG. 9, except that it further comprises fabric strips 536a, 536b, 538a, and 538b arranged at inner sides (e.g., edges) of the folds formed between the portions of the commissure tabs 508a and 508b. The fabric strips 536a, 536b, 538a, and 538b can further reduce abrasion and/or degradation of the commissure tabs at locations where the fasteners are positioned (e.g., the fabric strips are positioned between and separate an outer surface of the commissure tabs and ends of one or more fasteners).

For example, for each commissure tab 508a, 508b, a first fabric strip 536a, 536b can be provided at the respective fold formed between the second portion 512a, 512b and the third portion 514a, 514b, to provide further protection from abrasion to the fold region through which the third fastener 534a, 534b extends. Similarly, for each commissure tab 508a, 508b, a second fabric strip 538a, 538b can be provided at the respective fold formed between the third portion 514a, 514b and the fourth portion 515a, 515b, to provide further protection from abrasion to the fold region through which the second fastener 528a, 528b extends.

FIG. 12 shows the second embodiment of the commissure 502, as shown in FIGS. 9 and 10, attached to an alternate attachment member. In FIG. 12, the commissure 502 is secured to a wire-form attachment member 600 comprising a unitary wire-form body 602.

An exemplary embodiment of the wire-form attachment member 600 is shown in FIG. 11. The wire-form body 602 includes first and second coupling arms 612 and 614 that are configured to slide into and be retained within corresponding first and second channels 604 and 606 of a commissure support portion 622 of a frame of the prosthetic valve (e.g., channels in an actuator of the frame).

The wire-form body 602 can further include a front body 630, arranged radially inward of and offset from the first and second coupling arms 612 and 614, the front body 630 comprising first and second axially-extending members 608 and 610. The first coupling arm 612 can be coupled to the first axially-extending member 608 by a first curved member 616 and the second coupling arm 614 can be coupled to the second axially-extending member 610 by a second curved member 618. Further, the first and second axially-extending members 608 and 610 can be coupled together by a member 620 which can be curved or straight. Thus, as shown in FIG. 11, the front body 630, between the first and second axially-extending members 608 and 610, forms an open frame.

In alternate embodiments, the front body 630 of the wire-form body 602 may have alternate shapes. For example, instead of two axially-extending members 608 and 610, the front body 630 may comprise a single, axially-extending member coupled to each of the first and second coupling arms 612 and 614.

As shown in FIG. 12, the commissure 502 may be coupled to the wire-form attachment member 600 in a similar manner to that shown in FIGS. 9 and 10, as described above. For example, the second fasteners 528a and 528b may attach the same portions of the commissure tabs 508a and 508b (as shown in FIGS. 9 and 10) to a portion of the wire-form attachment member 600 (e.g., secured around a portion of the front body 630, such as one of the axially-extending members 608 and 610), the third fasteners 530a and 530b may attach the same portions of the commissure tabs 508a and 508b to another portion of the wire-form attachment member 600 (e.g., secured around a portion of the front body 630, such as one of the first and second curved members 616 and 618), and the fourth fasteners 534a and 534b may attach the free ends 532a and 532b of the commissure tabs to a portion of the wire-form attachment member 600 (e.g., secured around a portion of the front body 630, such as one of the first and second curved members 616 and 618).

As shown in FIG. 12, the first and second coupling arms 612 and 614 are inserted into respective first and second channels 604 and 606 of the commissure support portion 622 of the frame of the prosthetic heart valve.

In this way, the folded commissure tabs 508a and 508b of the second embodiment shown in FIGS. 9, 10, and 12 are configured to offset the bending regions of the leaflets 504a and 504b (e.g., main bodies 506a and 506b), which are the load carrying regions of the leaflets, away from the commissure support portions 518 of the frame or attachment members attached thereto (e.g., attachment member 516 or wire-form attachment member 600). As a result, the movable portions of the leaflets do not contact the commissure support portion 518 or components attached thereto. Further, the folded commissure tab portions together form a "virtual window", having the leaflets and their movable or bendable regions, positioned therein. For example, each folded commissure tab is arranged outward of, in a lateral direction arranged tangent to a circumference of the prosthetic heart valve, a portion of the main body of the leaflet that is directly connected to the commissure tab. Thus, during operation of the prosthetic heart valve, the movable portions of the leaflets are maintained interior to folded commissure tabs (in the lateral direction) and do not come into contact with the commissure support portion of the frame or the attachment member.

Additionally, the fasteners (e.g., sutures) used to attach the commissure 502 to the attachment member and/or commissure support portion of the frame extend through the tab portions and their folds, and do not penetrate the main bodies of the leaflets, including their movable or bendable regions. This further increases the stability and longevity of the commissure assembly (e.g., the leaflets experience reduced stress and the fasteners experience less wear). Further, in comparison to the commissure assembly 300 of FIGS. 6 and 8, the commissure assembly 500 of FIGS. 9, 10, and 12 may be more robust and subject to less wear due to the increased number of attachments (e.g., fasteners) between the commissure and the attachment member and/or commissure support portion of the frame and due to at least one set of fasteners (second fasteners 528a and 528b) extending through two layers of the folded commissure tabs 508a and 508b (instead of only one).

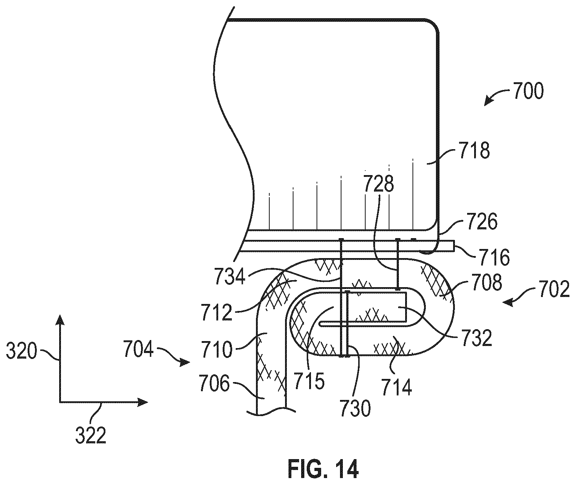

FIGS. 13 and 14 show a third embodiment of a commissure assembly 700 for a prosthetic heart valve. The prosthetic heart valve can be one of the prosthetic heart valves 10 (FIG. 1) or 100 (FIGS. 2-3) disclosed herein. The commissure assembly 700 comprises a half commissure 702 formed from a first commissure tab 708 of a first leaflet 504. The folded, half commissure 702 is secured to an attachment member 716 (which is then secured to a commissure support portion 718 of a frame of the prosthetic heart valve, as described herein, via a fastener 726) or secured directly to the commissure support portion 718 (without the attachment member 716). The commissure support portion 718 may be the same or similar to the commissure support portion 318 of FIGS. 6 and 8 and/or the commissure support portion 518 of FIGS. 9, 10, and 12, as described above. The half commissure 702 can be paired with another half commissure formed from a commissure tab of an adjacent leaflet, to form a commissure secured to the attachment member 716 and/or commissure support portion 718.

Thus, though FIGS. 13 and 14 only show half of the commissure (one folded commissure tab), it should be noted that a second commissure tab can be folded and secured in a same manner as described below for the first commissure tab 708 (e.g., the folded second commissure tab may be a mirror image of the first commissure tab 708 shown in FIGS. 13 and 14 and positioned directly adjacent the first commissure tab 708, similar to as shown in the embodiments of FIGS. 6, 8-10, and 12). However, unlike the other embodiments described above, in the embodiment of FIGS. 13 and 14, the two half commissures may be folded and secured to the attachment member separately and may not be secured to one another. Instead, the two half commissures of the adjacently arranged leaflets may be arranged adjacent to one another, and through the individual securing described further below, may be held together to form a commissure, without additional fasteners connecting the two half commissures (such as fastener 324 shown in FIGS. 6 and 8 and fastener 524 shown in FIGS. 9, 10, and 12).

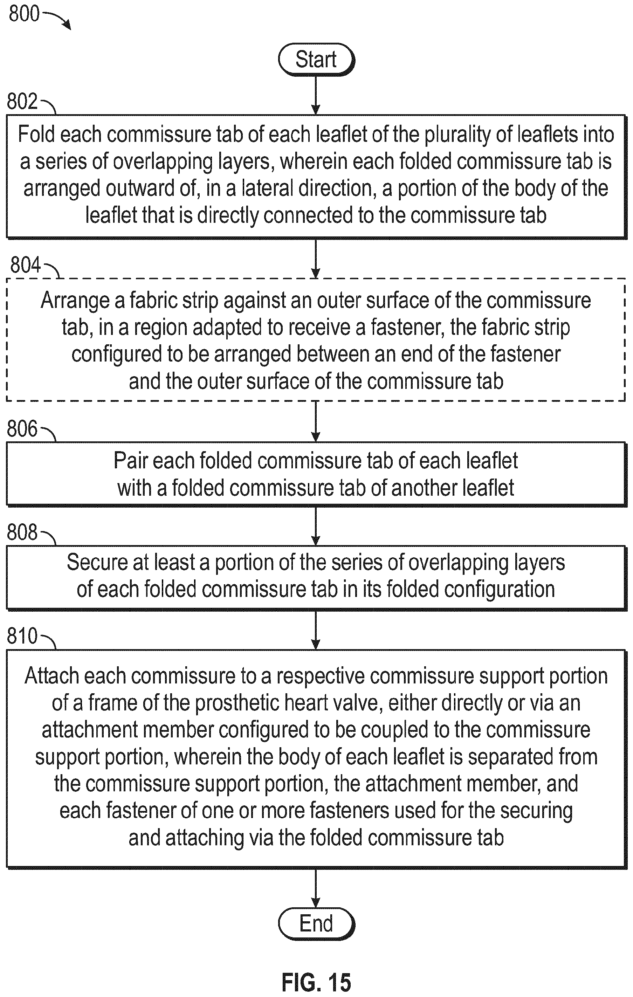

As explained above with reference to FIGS. 6 and 8, each leaflet includes two opposing commissure tabs arranged on opposite sides of a main body of the leaflet (only one commissure tab on one side of the main body of each leaflet of the half commissure 702 is shown in FIGS. 14 and 15). Further, as introduced above and described further below, each commissure tab may be folded and secured to the attachment member 716 (or commissure support portion 718) individually (without having to be first attached to an adjacent half commissure). This increases the ease of assembly and manufacturing of the commissure assembly.

FIGS. 13 and 14 show the commissure assembly 700 in two stages of assembly. As described further below, each half commissure 702 of a valve leaflet assembly is formed by folding the commissure tab 708 inward and over itself, such that the outermost, free end 732 of the commissure tab 708 is hidden and arranged within (e.g., within an interior of) the folds, thereby preventing the free end 732 from interfering with the motion of the main body 706 of the corresponding leaflet 704. The free end 732 of the commissure tab 708 is arranged at an opposite end of the commissure tab 708 from an end that is directly connected and continuous with the main body 706 of the leaflet 704.

Turning first to FIG. 13, a first stage of assembling the half commissure 702 is shown. The commissure tab 708 comprises a first portion 710 that is directly connected to and extends continuously and radially outward (relative to the radial direction 320) from the main body 706. The first portion 710 can further extend toward the attachment member 716 (which may be the same or similar to the attachment member 316 described above with reference to FIGS. 6 and 8 and/or the attachment member 516 described above with reference to FIGS. 9, 10, and 12) and/or commissure support portion 718 of the frame of the prosthetic heart valve. The commissure tab 708 is then folded over from the first portion 710 (e.g., at, approximately, a 90 degree angle) to extend in the lateral direction 322, away from the first portion 710, to form a second portion 712. As shown in FIG. 13, the second portion 712 extends substantially parallel to an inner surface of the commissure support portion 718 of the frame when attached (either directly or indirectly) thereto. The commissure tab 708 is then folded over from the second portion 718 (e.g., at, approximately, a 90 degree angle) to form a third portion 714 which extends radially inward, away from the attachment member 716. The third portion 714 is shown in a temporary orientation in FIG. 13 (e.g., during the folding process which is not its final orientation) which is approximately perpendicular to the attachment member 716.

The commissure tab 708 is then further folded over itself (folded over the third portion 714) to form a fourth portion 715 which extends radially outward, toward the second portion 712. The fourth portion is shown in a temporary orientation in FIG. 13 (e.g., during the folding process) which is approximately perpendicular to the attachment member 716 and the second portion 712.

As shown in FIG. 13, two fasteners can then be used to attach the partially folded commissure tab 708 to the attachment member 716 and secure an end of the commissure tab 708 in its partially folded configuration.

For example, a first fastener 728 can extend from an inner edge of the second portion 712, through the second portion 712 and to the attachment member 716. In some embodiments, the first fastener 728 may extend through the attachment member 716 (e.g., through a fabric strip or through an aperture in a plate-type attachment member) or around the attachment member 716 (e.g., around a portion of a wire-form attachment member) to secure the partially folded commissure tab 708 to the attachment member 716.

A second fastener 730 can extend from an outer edge of the third portion 714, through each of the third portion 714 and the fourth portion 715, and to an outer edge of the fourth portion 715 to secure the third portion 714 and the fourth portion 715 to one another, in their folded and overlapping configuration.

In a second stage of assembling the half commissure 702, as shown in FIG. 14, the third portion 714 and fourth portion 715, which are secured to each other via the second fastener 730 and thus move together as a single portion, are then folded inwardly, toward the first portion 710 and second portion 712. As a result, in this final, inwardly folded orientation, a majority portion (e.g., relatively straight portion arranged between the folds or bends) of each of the second portion 712, the third portion 714, and the fourth portion 715 are arranged substantially parallel to each other. In this way, the majority portion of each of the second portion 712, the third portion 714, and the fourth portion 715 overlap one another in the radial direction 320.

Further, as shown in FIG. 14, the fourth portion 715 is arranged interior to the other portions of the commissure tab 708, thereby making it "hidden". More specifically, the fourth portion 715 can be arranged between the second portion 712 and the third portion 714 and the free end 732 can be tucked inside the folds of the folded commissure tab 708, such that the free end 732 cannot contact and interfere with motion of the main body 706, during operation of the prosthetic heart valve. Due to this inward folding method, additional fasteners are not needed to secure the free end 732 away from the main body 706.

As shown in FIG. 14, a third fastener 734 can extend from an outer edge of the third portion 714, through each of the third portion 714, the fourth portion 715, and the second portion 712, and to (and through) the attachment member 716.

In some embodiments, the third fastener 734 can be arranged directly adjacent to the second fastener 730. In other embodiments, the third fastener 734 and second fastener 730 may overlap one another, along the radial direction 320.

In some embodiments, as shown in FIG. 14, the second fastener 730 and the third fastener 734 are arranged closer to the first portion 710 than the first fastener 728.

In some embodiments, the first fastener 728, second fastener 730, and/or third fastener 734 can be sutures.

In this way, the resulting half commissure 702 hides the inwardly folded end portion (e.g., free end 732) of the commissure tab 707, thereby mitigating interference with the motion of the working areas (e.g., main body 706) of the leaflet 704.

Further, the above-described folding of the commissure tab 708 creates a bulky section that can exert a sufficient push force against the first portion 710 and the main body 706 of the leaflet 704, thereby holding the leaflet 704 tightly against an adjacently arranged second leaflet (not shown in FIGS. 13 and 14). As a result, V-shaped stitches (such as the V-shaped fasteners 528a and 528b shown in FIGS. 9, 10, and 12) are not needed and a connection between the adjacent commissure tabs of the adjacent leaflets (such as the first fastener 524 shown in FIGS. 9, 10, and 12) is also not needed. Thus, assembly of the commissure is simplified and can therefore take less time and effort (as compared to the other methods described herein, for example). Further, stitches that may extend through working (e.g., moving during valve operation) areas of the leaflets may be avoided by not utilizing any V-shaped stitches, thereby increasing the durability of the leaflet assembly.

The commissure assembly 700 shown in FIGS. 13 and 14 may additionally provide the advantages described above for the other commissure assembly embodiments. For example, the folding and attachment configuration of the commissure assembly 700 may increase the stability and longevity of the leaflet assembly of the prosthetic heart valve, due to separating the working portions (e.g., main bodies) of the leaflets from the attachment member and/or commissure support portion of the frame, and fasteners (e.g., sutures) used to secure the commissure to the frame.

FIG. 15 shows a flow chart of a method 800 for assembling a prosthetic heart valve comprising a plurality of leaflets. The prosthetic heart valve can be one of the prosthetic heart valves disclosed herein, such as prosthetic heart valve 10 of FIG. 1 or prosthetic heart valve 100 of FIGS. 2 and 3. As described above, the prosthetic heart valve can comprise a frame with a plurality of leaflets mounted therein. In some embodiments, the prosthetic heart valve can include three leaflets. In other embodiments the prosthetic heart valve can include more or less than three leaflets (e.g., two leaflets). As described above, each leaflet includes two opposing commissure tabs arranged on opposite sides of a body (referred to herein as a main body) of the leaflet, the body configured to move during operation of the prosthetic heart valve (e.g., when implanted in a heart of a patient). Specifically, the body of each leaflet can be referred to as a working portion of the leaflet which is movable during operation of the prosthetic heart valve and each folded commissure tab can be immobile relative to the corresponding body, when secured to a respective attachment member and/or commissure support portion of the frame of the prosthetic heart valve.

As used herein, a lateral direction and radial direction may be relative to a central longitudinal axis of a frame of the prosthetic heart valve and a circumference of the frame and/or valve. For example, the lateral direction is arranged tangent to the circumference of the prosthetic heart valve and the radial direction extends outward from the central longitudinal axis, toward the circumference.

Method 800 begins at 802 and includes folding each commissure tab of each leaflet of the plurality of leaflets into a series of overlapping layers, wherein each folded commissure tab is arranged outward of, in a lateral direction arranged tangent to a circumference of the prosthetic heart valve, a portion of the body of the leaflet that is directly connected to the commissure tab.

In a first embodiment, the folding at 802 can include folding each commissure tab so that a first portion of the commissure tab, the first portion directly connected to the body of the corresponding leaflet, extends radially outward from the body, a second portion is bent over the first portion and extends radially inward and toward the body, and a third portion is bent over the second portion and extends radially outward and away from the body, where a majority of each of the first, second, and third portions are arranged in parallel with one another and overlap in the lateral direction. An example commissure resulting from the folding in the first embodiment is shown in FIGS. 6 and 8, as described above.

In a second embodiment, the folding at 802 can include folding each commissure tab so that a first portion of the commissure tab, the first portion directly connected to the body of the corresponding leaflet, extends radially outward from the body, a second portion is bent away from the first portion and extends laterally outward, in the lateral direction, from the first portion, a third portion is folded over the second portion and extends laterally inward, and a fourth portion is folded over the third portion and extends laterally outward, where a majority of each of the second, third, and fourth portions are arranged in parallel with one another and overlap in a radial direction. An example commissure resulting from the folding in the second embodiment is shown in FIGS. 9, 10, and 12, as described above.

In a third embodiment, the folding at 802 can include folding each commissure tab so that a first portion of the commissure tab, the first portion directly connected to the body of the corresponding leaflet, extends radially outward, in the lateral direction, from the body, a second portion is bent away from the first portion and extends laterally outward from the first portion, a third portion is bent around from the second portion and extends laterally inward, and a fourth portion is folded over the third portion and extends laterally outward, where the fourth portion is arranged between the second and third portions, in the radial direction, and where a majority of each of the second, third, and fourth portions are arranged in parallel with one another and overlap in the radial direction. An example half commissure resulting from the folding in the third embodiment is shown in FIG. 14.

The method 800 optionally includes, at 804, arranging a fabric strip against an outer surface of the commissure tab in a region adapted to receive a fastener, the fabric strip configured to be arranged between an end of the fastener and the outer surface of the commissure tab. An example of such a fabric strip is shown in FIGS. 10 and 12 (e.g., fabric strips 536a, 536b, 538a, and 538b). In other embodiments, the fabric strip may be comprised of a different material, other than fabric, such as a flexible polymer.

Continuing to 806, method 800 includes pairing each folded commissure tab of each leaflet with a folded commissure tab of another (adjacent) leaflet.

At 808, method 800 includes securing at least a portion of the series of overlapping layers of each folded commissure tab in its folded configuration.

In the first embodiment, the securing at 808 can include, for each pair of folded commissure tabs, wrapping a band (e.g., fastener 324 shown in FIG. 6) around an outer edge of the second portion of a first folded commissure tab of the pair of folded commissure tabs to and around an outer edge of the second portion of a second folded commissure tab of the pair of commissure tabs, to secure the first and second folded commissure tabs to one another.

In the second embodiment, the securing at 808 can include, for each pair of folded commissure tabs, wrapping a band (e.g., fastener 524 shown in FIG. 9) around an inner edge of a bend between the third and fourth portions of a first folded commissure tab of the pair of folded commissure tabs to and around an inner edge of a bend between the third and fourth portions of a second folded commissure tab of the pair of commissure tabs, to secure the first and second folded commissure tabs to one another.

In the third embodiment, the securing at 808 can include extending a fastener (e.g., fastener 730 shown in FIGS. 13 and 14) from an outer edge of the third portion, through the third and fourth portions, and to an outer edge of the fourth portion.

Together, the methods at 802, 804 (optionally), 806, and 808 result in the formation of a commissure of the prosthetic heart valve. Thus, the methods at 802, 804, 806, and 808 may be repeated for each pair of commissure tabs of a valve leaflet assembly in order to form all the commissures of the prosthetic heart valve. For example, for a valve including three leaflets, three commissures may be formed.

After forming each commissure of the prosthetic heart valve, method 800 continues to 810 and includes, for each commissure, attaching the commissure to a respective commissure support portion of a frame of the prosthetic valve, either directly or via an attachment member configured to be coupled to the commissure support portion, wherein the body of each leaflet is separated from the commissure support portion, the attachment member, and each fastener of one or more fasteners used for the securing and attaching via the folded commissure tab.

In the first embodiment, the attaching at 810 can include, for each folded commissure tab, extending a first fastener (e.g., fasteners 328a and 328b shown in FIG. 6) from an inner edge of a bend between the first portion and the second portion to the commissure support portion or the attachment member to secure the folded commissure tab to the commissure support portion or the attachment member. In some embodiments, the attaching at 810 can further include securing a terminal, free end of each folded commissure tab to the commissure support portion or attachment member via a second fastener (e.g., fasteners 330a and 330b shown in FIG. 6).

In the second embodiment, the attaching at 810 can include, for each folded commissure tab, extending a first fastener (e.g., fasteners 528a and 528b shown in FIG. 9) from an inner edge of a bend between the third portion and the fourth portion to the commissure support portion or the attachment member to secure the folded commissure tab to the commissure support portion or the attachment member. In some embodiments, the attaching at 810 can further include, for each folded commissure tab, extending a second fastener (e.g., fasteners 530a and 530b shown in FIG. 9) from an inner edge of a bend between the second portion and the third portion to the commissure support portion or the attachment member to secure the folded commissure tab to the commissure support portion or the attachment member. In some embodiments, the attaching at 810 can further include securing a terminal, free end of each folded commissure tab to the commissure support portion or the attachment member via a third fastener (e.g., fasteners 534a and 534b shown in FIG. 9.

In the third embodiment, the attaching at 810 can include, for each commissure tab, extending a fastener (e.g., fastener 728 shown in FIGS. 13 and 14) from an inner edge of the second portion, through the second portion, and to the commissure support portion or attachment member and extending another fastener (e.g., fastener 734 shown in FIG. 14) from an outer edge of the third portion, through each of the third portion, the fourth portion, and the second portion, and to the commissure support portion or attachment member.

In some embodiments, the attaching at 810 can further include securing the attachment member to the commissure support portion via an additional fastener.

In this way, commissures of a prosthetic heart valve may be formed and attached to a commissure support portion of a frame of the prosthetic heart valve in a manner that increases the stability and longevity of the leaflet assembly. For example, as discussed above, the fasteners and leaflets of the commissures may experience reduced wear over time.

### Additional Examples of the Disclosed Technology

In view of the above described implementations of the disclosed subject matter, this application discloses the additional examples enumerated below. It should be noted that one feature of an example in isolation or more than one feature of the example taken in combination and, optionally, in combination with one or more features of one or more further examples are further examples also falling within the disclosure of this application.

Example 1. A method of assembling a prosthetic heart valve comprising a plurality of leaflets, comprising: forming a plurality of commissures with the plurality of leaflets, wherein each commissure is formed by: folding each commissure tab of each leaflet of the plurality of leaflets into a series of overlapping layers, wherein each leaflet includes two opposing commissure tabs arranged on opposite sides of a body of the leaflet, the body configured to move during operation of the prosthetic heart valve, wherein each folded commissure tab is arranged outward of, in a lateral direction arranged tangent to a circumference of the prosthetic heart valve, a portion of the body of the leaflet that is directly connected to the commissure tab; pairing each folded commissure tab of each leaflet with a folded commissure tab of another leaflet; and securing at least a portion of the series of overlapping layers of each folded commissure tab in its folded configuration; and for each commissure, attaching the commissure to a respective commissure support portion of a frame of the prosthetic heart valve, either directly or via an attachment member configured to be coupled to the commissure support portion, wherein the body of each leaflet is separated from the commissure support portion, the attachment member, and each fastener of one or more fasteners used for the securing and attaching via the folded commissure tab.

Example 2. The method of any example herein, particularly example 1, wherein the body of each leaflet is a working portion of the leaflet which is movable during operation of the prosthetic heart valve and wherein each folded commissure tab is immobile relative to the corresponding body.

Example 3. The method of any example herein, particularly any one of examples 1-2, wherein the folding includes folding each commissure tab so that a first portion of the commissure tab, the first portion directly connected to the body of the corresponding leaflet, extends radially outward from the body, a second portion is bent over the first portion and extends radially inward and toward the body, and a third portion is bent over the second portion and extends radially outward and away from the body, wherein a majority of each of the first, second, and third portions are arranged in parallel with one another and overlap in the lateral direction.

Example 4. The method of any example herein, particularly example 3, wherein the securing includes, for each pair of folded commissure tabs, wrapping a band around an outer edge of the second portion of a first folded commissure tab of the pair of folded commissure tabs to and around an outer edge of the second portion of a second folded commissure tab of the pair of commissure tabs, to secure the first and second folded commissure tabs to one another.

Example 5. The method of any example herein, particularly any one of examples 3-4, wherein the attaching includes, for each folded commissure tab, extending a first fastener from an inner edge of a bend between the first portion and the second portion to the commissure support portion or the attachment member to secure the folded commissure tab to the commissure support portion or the attachment member.

Example 6. The method of any example herein, particularly any one of examples 3-5, wherein the attaching includes securing a terminal, free end of each folded commissure tab to the commissure support portion or attachment member via a second fastener.

Example 7. The method of any example herein, particularly any one of examples 1-2, wherein the folding includes folding each commissure tab so that a first portion of the commissure tab, the first portion directly connected to the body of the corresponding leaflet, extends radially outward from the body, a second portion is bent away from the first portion and extends laterally outward, in the lateral direction, from the first portion, a third portion is folded over the second portion and extends laterally inward, and a fourth portion is folded over the third portion and extends laterally outward, wherein a majority of each of the second, third, and fourth portions are arranged in parallel with one another and overlap in a radial direction.

Example 8. The method of any example herein, particularly example 7, wherein the securing includes, for each pair of folded commissure tabs, wrapping a band around an inner edge of a bend between the third and fourth portions of a first folded commissure tab of the pair of folded commissure tabs to and around an inner edge of a bend between the third and fourth portions of a second folded commissure tab of the pair of commissure tabs, to secure the first and second folded commissure tabs to one another.

Example 9. The method of any example herein, particularly any one of examples 7-8, wherein the attaching includes, for each folded commissure tab, extending a first fastener from an inner edge of a bend between the third portion and the fourth portion to the commissure support portion or the attachment member to secure the folded commissure tab to the commissure support portion or the attachment member.

Example 10. The method of any example herein, particularly any one of examples 7-9, wherein the attaching includes, for each folded commissure tab, extending a second fastener from an inner edge of a bend between the second portion and the third portion to the commissure support portion or the attachment member to secure the folded commissure tab to the commissure support portion or the attachment member.

Example 11. The method of any example herein, particularly any one of examples 7-10, wherein the attaching includes securing a terminal, free end of each folded commissure tab to the commissure support portion or the attachment member via a third fastener.

Example 12. The method of any example herein, particularly any one of examples 7-11, further comprising arranging a fabric strip against an outer surface of the commissure tab in a region through which a fastener extends, the fabric strip arranged between an end of the fastener and the outer surface of the commissure tab.

Example 13. The method of any example herein, particularly any one of examples 1-2, wherein the folding includes folding each commissure tab so that a first portion of the commissure tab, the first portion directly connected to the body of the corresponding leaflet, extends radially outward, in the lateral direction, from the body, a second portion is bent away from the first portion and extends laterally outward from the first portion, a third portion is bent around from the second portion and extends laterally inward, and a fourth portion is folded over the third portion and extends laterally outward, wherein the fourth portion is arranged between the second and third portions, in the radial direction, and wherein a majority of each of the second, third, and fourth portions are arranged in parallel with one another and overlap in the radial direction.

Example 14. The method of any example herein, particularly example 13, wherein the securing and attaching includes, for each commissure tab, extending a first fastener from an inner edge of the second portion, through the second portion, and to the commissure support portion or attachment member, extending a second fastener from an outer edge of the third portion, through the third and fourth portions, and to an outer edge of the fourth portion, and extending a third fastener from an outer edge of the third portion, through each of the third portion, the fourth portion, and the second portion, and to the commissure support portion or attachment member.

Example 15. The method of any example herein, particularly example 14, wherein the securing and attaching via the first fastener and the second fastener occurs during the folding and the securing and attaching via the third fastener occurs after the folding is complete.

Example 16. The method of any example herein, particularly any one of the preceding examples, wherein attaching the folded commissure tabs of the leaflet assembly to the commissure support portion of the prosthetic heart valve or the attachment member configured to be coupled to the commissure support portion includes attaching the folded commissure tabs of the leaflet assembly to the attachment member and further comprising securing the attachment member to the commissure support portion via an additional fastener.

Example 17. The method of any example herein, particularly any one of the preceding examples, wherein the attachment member comprises a fabric material, a rigid plate, or a wire-form body.

Example 18. The method of any example herein, particularly any one of the preceding examples, wherein attaching the commissure to the respective commissure support portion of the frame of the prosthetic heart valve includes attaching the commissure to a commissure support portion of an actuator or an expansion and locking mechanism of the frame.

Example 19. The method of any example herein, particularly any one of the preceding examples, further comprising attaching an inflow edge of the body of each leaflet directly to an inner skirt of the prosthetic heart valve, the inner skirt mounted on an inner surface of the frame.

Example 20. The method of any example herein, particularly any one of the preceding examples, wherein the frame comprises a plurality of pivotably connected struts.

Example 21. A prosthetic heart valve, comprising: an annular frame comprising a plurality of commissure support portions; a plurality of attachment members, each secured to a corresponding support portion of the plurality of commissure support portions; and a plurality of leaflets, each leaflet comprising a body and two opposing commissure tabs arranged on opposite sides of the body, wherein each commissure tab is folded into a series of overlapping layers where majority portions of each overlapping layer are arranged in parallel with one another and overlap in a lateral direction, the lateral direction arranged tangent to a circumference of the annular frame, wherein each folded commissure tab is arranged adjacent to another folded commissure tab of an adjacent leaflet to form a pair of commissure tabs forming a commissure, and wherein each commissure is coupled to a corresponding attachment member of the plurality of attachment members; and wherein for each commissure: a first fastener is wrapped around at least a portion of the series of overlapping layers of both commissure tabs of the pair of commissure tabs of the commissure to secure the pair of commissure tabs together; a second fastener extends between an interior fold of a first folded commissure tab of the pair of commissure tabs and the corresponding attachment member to secure the first folded commissure tab to the attachment member; a third fastener extends between an interior fold of a second folded commissure tab of the pair of commissure tabs and the corresponding attachment member to secure the second folded commissure tab to the attachment member; and wherein each of the first, second, and third fasteners are separated from the bodies of the leaflets to which the commissure tabs of the pair of commissure tabs are connected by folds of the commissure tabs.

Example 22. The prosthetic heart valve of any example herein, particularly example 21, wherein the series of overlapping layers of each folded commissure tab comprises: a first portion that is directly connected to the body and extends radially outward from the body and toward the attachment member and commissure support portion to which the folded commissure tab is coupled, a second portion that is folded over the first portion and extends radially inward and toward the body, and a third portion that is folded over the second portion and extends radially outward and toward the attachment member and commissure support portion, wherein a majority of each of the first, second, and third portions are arranged in parallel with one another and overlap in the lateral direction.

Example 23. The prosthetic heart valve of any example herein, particularly example 22, wherein the first fastener is wrapped around the folded portions of the pair of commissure tabs of the commissure, from an outer edge of the second portion of a first folded commissure tab of the pair of commissure tabs to an outer edge of the second portion of a second folded commissure tab of the pair of commissure tabs, to secure the first and second portions of each folded commissure tab of the pair of commissure tabs together.

Example 24. The prosthetic heart valve of any example herein, particularly any one of examples 22-23, wherein the second fastener extends from an inner edge of a bend between the first and second portions of the first folded commissure tab, through a thickness of the bend, and to the corresponding attachment member to secure the first folded commissure tab to the attachment member and wherein the third fastener extends from an inner edge of a bend between the first and second portion of the second folded commissure tab, through a thickness of the bend, and to the corresponding attachment member to secure the second folded commissure tab to the attachment member.

Example 25. The prosthetic heart valve of any of the preceding examples, wherein, for each commissure, an outermost, free end of the first folded commissure tab is secured to the attachment member via a fourth fastener and an outermost, free end of the second folded commissure tab is secured to the attachment member via a fifth fastener.

Example 26. The prosthetic heart valve of any of the preceding examples, wherein, for each commissure, a sixth fastener secures each attachment member to the corresponding commissure support portion and wherein the attachment member is one of a fabric strip, a rigid base plate, or a wire-form body configured to couple with one or more channels in the commissure support portion.

Example 27. The prosthetic heart valve of any of the preceding examples, further comprising an inner skirt mounted on an inner surface of the frame, wherein an inflow edge of the body of each leaflet is attached directly to the inner skirt and wherein the inner skirt is directly connected to struts of the frame.

Example 28. The prosthetic heart valve of any of the preceding examples, wherein the frame comprises a plurality of pivotably connected struts.

Example 29. The prosthetic heart valve of any of the preceding examples, wherein the frame comprises a plurality of expansion and locking mechanism configured to radially expand and lock the frame in a radially expanded state and wherein each expansion and locking mechanism includes one of the plurality of commissure support portions.

Example 30. A prosthetic heart valve, comprising: an annular frame comprising a plurality of commissure support portions; a plurality of attachment members, each secured to a corresponding support portion of the plurality of commissure support portions; and a plurality of leaflets, each leaflet comprising a body and two opposing commissure tabs arranged on opposite sides of the body, wherein each commissure tab is folded into a series of overlapping layers where majority portions of each overlapping layer are arranged in parallel with one another and overlap in a radial direction, relative to a central longitudinal axis of the prosthetic heart valve, wherein each folded commissure tab is arranged adjacent to another folded commissure tab of an adjacent leaflet to form a pair of commissure tabs forming a commissure, and wherein each commissure is coupled to a corresponding attachment member of the plurality of attachment members; and wherein for each commissure: a first fastener is wrapped around at least a portion of the series of overlapping layers of both commissure tabs of the pair of commissure tabs of the commissure to secure the pair of commissure tabs together; for each folded commissure tab of the pair of commissure tabs: a second fastener extends from a first interior fold of the folded commissure tab, through two overlapping layers of the series of overlapping layers, and to the corresponding attachment member to secure an inner portion, relative to a lateral direction arranged tangent to a circumference of the annular frame, of the folded commissure tab to the attachment member; and a third fastener extends from a second interior fold of the folded commissure tab, through one layer of the series of overlapping layers, and to the corresponding attachment member to secure an outer portion, relative to the lateral direction, of the folded commissure tab to the attachment member; and wherein each of the first, second, and third fasteners are separated from the bodies of the leaflets to which the commissure tabs of the pair of commissure tabs are connected by folds of the commissure tabs.

Example 31. The prosthetic heart valve of any example herein, particularly example 30, wherein each folded commissure tab comprises: a first portion that is directly connected to the body of the corresponding leaflet and extends radially outward from the body and toward the attachment member and commissure support portion to which the folded commissure tab is coupled, a second portion that is folded away from the first portion and extends laterally outward, in the lateral direction, from the first portion, a third portion that is folded over the second portion and extends laterally inward toward the first portion, and a fourth portion that is folded over the third portion and extends laterally outward and away from the first portion, wherein a majority of each of the second, third, and fourth portions are arranged in parallel with one another and overlap in the radial direction.

Example 32. The prosthetic heart valve of any example herein, particularly example 31, wherein the first fastener is wrapped around the folded portions of the pair of commissure tabs of the commissure, from an inner edge of a first bend between the third and fourth portions of a first folded commissure tab of the pair of commissure tabs, to an inner edge of a second bend between the third and fourth portions of a second folded commissure tab of the pair of commissure tabs, to secure the first and second commissure tabs together.

Example 33. The prosthetic heart valve of any example herein, particularly any one of examples 31-32, wherein the second fastener extends from an inner edge of a bend between the third and fourth portions of the folded commissure tab, through a thickness of each of the third portion and second portion, and to the corresponding attachment member to secure the folded commissure tab to the attachment member and wherein the third fastener extends from an inner edge of a bend between the second and third portions of the folded commissure tab, through a thickness of the second portion, and to the corresponding attachment member to further secure the folded commissure tab to the attachment member.

Example 34. The prosthetic heart valve of any of the preceding examples, wherein, for each commissure, an outermost, free end of each folded commissure tab is secured to the attachment member via a fourth fastener.

Example 35. The prosthetic heart valve of any of the preceding examples, wherein the attachment member is one of a fabric strip, a rigid base plate, or a wire-form body configured to couple with one or more channels in the commissure support portion.

Example 36. The prosthetic heart valve of any of the preceding examples, further comprising an inner skirt mounted on an inner surface of the frame, wherein an inflow edge of the body of each leaflet is attached directly to the inner skirt and wherein the inner skirt is directly connected to struts of the frame.

Example 37. The prosthetic heart valve of any of the preceding examples, wherein the frame comprises a plurality of pivotably connected struts.

Example 38. The prosthetic heart valve of any of the preceding examples, wherein the frame comprises a plurality of expansion and locking mechanism configured to radially expand and lock the frame in a radially expanded state and wherein each expansion and locking mechanism includes one of the plurality of commissure support portions.

Example 39. A prosthetic heart valve, comprising: an annular frame comprising a plurality of commissure support portions; a plurality of attachment members, each secured to a corresponding support portion of the plurality of commissure support portions; a plurality of leaflets, each leaflet comprising a body and two opposing commissure tabs arranged on opposite sides of the body, wherein each commissure tab is folded into a series of overlapping layers with a terminal, free end of the commissure tab enclosed within the series of overlapping layers, wherein majority portions of each overlapping layer are arranged in parallel with one another and overlap in a radial direction, relative to a longitudinal axis of the prosthetic heart valve, wherein each folded commissure tab is arranged adjacent to another folded commissure tab of an adjacent leaflet to form a pair of commissure tabs forming a commissure, and wherein each commissure is coupled to a corresponding attachment member of the plurality of attachment members; and wherein, for each folded commissure tab of each commissure, a plurality of fasteners secure inner and outer portions, relative to a lateral direction arranged tangent to a circumference of the annular frame, of the folded commissure tab to the corresponding attachment member, wherein each of the plurality of fasteners are separated from the body of the leaflet to which the folded commissure tab is connected by folds of the folded commissure tab.

Example 40. The prosthetic heart valve of any example herein, particularly example 39, wherein each folded commissure tab comprises: a first portion that is directly connected to the body of the corresponding leaflet and extends radially outward from the body and toward the attachment member and commissure support portion to which the folded commissure tab is coupled, a second portion that is folded away from the first portion and extends laterally outward, in the lateral direction, from the first portion, a third portion that is bent around from the second portion and extends laterally inward toward the first portion, and a fourth portion that is folded over the third portion and extends laterally outward and away from the first portion, wherein the fourth portion is arranged between the second and third portions, in the radial direction, and wherein a majority of each of the second, third, and fourth portions are arranged in parallel with one another and overlap in the radial direction.

Example 41. The prosthetic heart valve of any example herein, particularly example 40, wherein the plurality of fasteners comprise: a first fastener extending from an inner edge of the second portion, through the second portion, and to the attachment member; a second fastener extending from an outer edge of the third portion, through the third and fourth portions, and to an outer edge of the fourth portion; and a third fastener extending from an outer edge of the third portion, through each of the third portion, the fourth portion, and the second portion, and to the attachment member, wherein the second and third fasteners are arranged closer to the first portion than the first fastener.

Example 42. The prosthetic heart valve of any of the preceding examples, further comprising an inner skirt mounted on an inner surface of the frame, wherein an inflow edge of the body of each leaflet is attached directly to the inner skirt and wherein the inner skirt is directly connected to struts of the frame.

Example 43. The prosthetic heart valve of any of the preceding examples, wherein the frame comprises a plurality of pivotably connected struts.

Example 44. The prosthetic heart valve of any of the preceding examples, wherein the frame comprises a plurality of expansion and locking mechanism configured to radially expand and lock the frame in a radially expanded state and wherein each expansion and locking mechanism includes one of the plurality of commissure support portions.

Example 45. A method of assembling a prosthetic heart valve comprising a plurality of leaflets, comprising: forming a plurality of commissures with the plurality of leaflets, wherein each commissure is formed by: folding each commissure tab of each leaflet of the plurality of leaflets into a series of overlapping layers that overlap in a lateral direction, the lateral direction arranged tangent to a circumference of the prosthetic heart valve, wherein each leaflet includes two opposing commissure tabs arranged on opposite sides of a body of the leaflet, the body configured to move during operation of the prosthetic heart valve, wherein each folded commissure tab is arranged outward of, in the lateral direction and a radial direction, a portion of the body of the leaflet that is directly connected to the commissure tab, the radial direction relative to a central longitudinal axis of the prosthetic heart valve; pairing each folded commissure tab of each leaflet with a folded commissure tab of another leaflet; and securing at least a portion of the series of overlapping layers of each folded commissure tab in its folded configuration; and for each commissure, attaching the commissure to a respective commissure support portion of a frame of the prosthetic heart valve, either directly or via an attachment member configured to be coupled to the commissure support portion, wherein the body of each leaflet is separated, in the radial direction, from the commissure support portion, the attachment member, and each fastener of one or more fasteners used for the securing and attaching via the folded commissure tab.

Example 46. The method of any example herein, particularly example 45, wherein the series of overlapping layers includes at least three overlapping layers.

Example 47. The method of any example herein, particularly example 46, wherein the folding includes folding each commissure tab so that a first portion of the commissure tab, the first portion directly connected to the body of the corresponding leaflet, extends radially outward from the body, a second portion is bent over the first portion and extends radially inward and toward the body, and a third portion is bent over the second portion and extends radially outward and away from the body, wherein a majority of each of the first, second, and third portions are arranged in parallel with one another.

Example 48. The method of any example herein, particularly example 47, wherein the securing includes, for each pair of folded commissure tabs, wrapping a band around an outer edge of the second portion of a first folded commissure tab of the pair of folded commissure tabs to and around an outer edge of the second portion of a second folded commissure tab of the pair of commissure tabs, to secure the first and second folded commissure tabs to one another.

Example 49. The method of any example herein, particularly any one of examples 47-48, wherein the attaching includes, for each folded commissure tab, extending a first fastener from an inner edge of a bend between the first portion and the second portion to the commissure support portion or the attachment member to secure the folded commissure tab to the commissure support portion or the attachment member.

Example 50. The method of any example herein, particularly any one of examples 47-49, wherein the attaching includes securing a terminal, free end of each folded commissure tab to the commissure support portion or attachment member via a second fastener.

Example 51. The method of any of the preceding examples, wherein the body of each leaflet is a working portion of the leaflet which is movable during operation of the prosthetic heart valve and wherein each folded commissure tab is immobile relative to the corresponding body.

Example 52. A method of assembling a prosthetic heart valve comprising a plurality of leaflets, comprising: forming a plurality of commissures with the plurality of leaflets, wherein each commissure is formed by: folding each commissure tab of each leaflet of the plurality of leaflets into a series of overlapping layers that wind consecutively and overlap in a radial direction, relative to a central longitudinal axis of the prosthetic heart valve, wherein a free end of each commissure tab is arranged exterior to folds of the folded commissure tab, wherein each leaflet includes two opposing commissure tabs arranged on opposite sides of a body of the leaflet, the body configured to move during operation of the prosthetic heart valve, wherein each folded commissure tab is arranged outward of, in a lateral direction arranged tangent to a circumference of the prosthetic heart valve and the radial direction, a portion of the body of the leaflet that is directly connected to the commissure tab; pairing each folded commissure tab of each leaflet with a folded commissure tab of another leaflet; and securing at least a portion of the series of overlapping layers of each folded commissure tab in its folded configuration; and for each commissure, attaching the commissure to a respective commissure support portion of a frame of the prosthetic heart valve, either directly or via an attachment member configured to be coupled to the commissure support portion, wherein the body of each leaflet is separated from the commissure support portion, the attachment member, and each fastener of one or more fasteners used for the securing and attaching via the folded commissure tab.

Example 53. The method of any example herein, particularly example 52, wherein the series of overlapping layers includes at least three overlapping layers.

Example 54. The method of any example herein, particularly example 53, wherein the folding includes folding each commissure tab so that a first portion of the commissure tab, the first portion directly connected to the body of the corresponding leaflet, extends radially outward from the body, a second portion is bent away from the first portion and extends laterally outward, in the lateral direction, from the first portion, a third portion is folded over the second portion and extends laterally inward, and a fourth portion is folded over the third portion and extends laterally outward, wherein a majority of each of the second, third, and fourth portions are arranged in parallel with one another.

Example 55. The method of any example herein, particularly example 54, wherein the securing includes, for each pair of folded commissure tabs, wrapping a band around an inner edge of a bend between the third and fourth portions of a first folded commissure tab of the pair of folded commissure tabs to and around an inner edge of a bend between the third and fourth portions of a second folded commissure tab of the pair of commissure tabs, to secure the first and second folded commissure tabs to one another.

Example 56. The method of any example herein, particularly any one of examples 54-55, wherein the attaching includes, for each folded commissure tab, extending a first fastener from an inner edge of a bend between the third portion and the fourth portion to the commissure support portion or the attachment member to secure the folded commissure tab to the commissure support portion or the attachment member.

Example 57. The method of any example herein, particularly any one of examples 54-56, wherein the attaching includes, for each folded commissure tab, extending a second fastener from an inner edge of a bend between the second portion and the third portion to the commissure support portion or the attachment member to secure the folded commissure tab to the commissure support portion or the attachment member.

Example 58. The method of any example herein, particularly any one of examples 54-57, wherein the attaching includes securing the free end of each folded commissure tab to the commissure support portion or the attachment member via a third fastener.

Example 59. The method of any example herein, particularly any one of examples 54-58, further comprising arranging a fabric strip against an outer surface of the commissure tab in a region through which a fastener extends, the fabric strip arranged between an end of the fastener and the outer surface of the commissure tab.

Example 60. The method of any example herein, wherein the body of each leaflet is a working portion of the leaflet which is movable during operation of the prosthetic heart valve and wherein each folded commissure tab is immobile relative to the corresponding body.

Example 61. A method of assembling a prosthetic heart valve comprising a plurality of leaflets, comprising: forming a plurality of commissures with the plurality of leaflets, wherein each commissure is formed by: folding each commissure tab of each leaflet of the plurality of leaflets into a series of overlapping layers that overlap in a radial direction, relative to a central longitudinal axis of the prosthetic heart valve, by folding the commissure tab inward and over itself, such that an outermost, free end of the commissure tab is arranged within an interior of folds of the folded commissure tab, wherein each leaflet includes two opposing commissure tabs arranged on opposite sides of a body of the leaflet, the body configured to move during operation of the prosthetic heart valve, and wherein each folded commissure tab is arranged outward of, in a lateral direction arranged tangent to a circumference of the prosthetic heart valve and the radial direction, a portion of the body of the leaflet that is directly connected to the commissure tab; pairing each folded commissure tab of each leaflet with a folded commissure tab of another leaflet; and securing at least a portion of the series of overlapping layers of each folded commissure tab in its folded configuration; and for each commissure, attaching the commissure to a respective commissure support portion of a frame of the prosthetic heart valve, either directly or via an attachment member configured to be coupled to the commissure support portion, wherein the body of each leaflet is separated from the commissure support portion, the attachment member, and each fastener of one or more fasteners used for the securing and attaching via the folded commissure tab.

Example 62. The method of any example herein, particularly example 61, wherein the series of overlapping layers includes at least three overlapping layers.

Example 63. The method of any example herein, particularly example 62, wherein the folding includes folding each commissure tab so that a first portion of the commissure tab, the first portion directly connected to the body of the corresponding leaflet, extends radially outward, in the lateral direction, from the body, a second portion is bent away from the first portion and extends laterally outward from the first portion, a third portion is bent around from the second portion and extends laterally inward, and a fourth portion is folded over the third portion and extends laterally outward, wherein the fourth portion is arranged between the second and third portions, in the radial direction, and wherein a majority of each of the second, third, and fourth portions are arranged in parallel with one another.

Example 64. The method of any example herein, particularly example 63, wherein the securing and attaching includes, for each commissure tab, extending a first fastener from an inner edge of the second portion, through the second portion, and to the commissure support portion or attachment member, extending a second fastener from an outer edge of the third portion, through the third and fourth portions, and to an outer edge of the fourth portion, and extending a third fastener from an outer edge of the third portion, through each of the third portion, the fourth portion, and the second portion, and to the commissure support portion or attachment member.

Example 65. The method of any example herein, particularly example 64, wherein the securing and attaching via the first fastener and the second fastener occurs during the folding and the securing and attaching via the third fastener occurs after the folding is complete.

Example 66. The method of any of the preceding examples, wherein the body of each leaflet is a working portion of the leaflet which is movable during operation of the prosthetic heart valve and wherein each folded commissure tab is immobile relative to the corresponding body.

Example 67. A prosthetic heart valve, comprising: an annular frame comprising a plurality of commissure support portions; a plurality of attachment members, each secured to a corresponding support portion of the plurality of commissure support portions; a plurality of leaflets, each leaflet comprising a body and two opposing commissure tabs arranged on opposite sides of the body, wherein each commissure tab is folded into a series of overlapping layers including at least three layers, wherein majority portions of each overlapping layer are arranged in parallel with one another, wherein each folded commissure tab is arranged adjacent to another folded commissure tab of an adjacent leaflet to form a pair of commissure tabs forming a commissure, and wherein each commissure is coupled to a corresponding attachment member of the plurality of attachment members; and wherein, for each folded commissure tab of each commissure, a plurality of fasteners secure inner and outer portions, relative to a lateral direction arranged tangent to a circumference of the annular frame, of the folded commissure tab to the corresponding attachment member, wherein each of the plurality of fasteners are separated from the body of the leaflet to which the folded commissure tab is connected by folds of the folded commissure tab.

Example 68. The prosthetic heart valve of any example herein, particularly example 67, wherein the majority portions of each overlapping layer overlap in the lateral direction.

Example 69. The prosthetic heart valve of any example herein, particularly example 68, wherein the plurality of fasteners includes: a first fastener that is wrapped around at least a portion of the series of overlapping layers of both commissure tabs of the pair of commissure tabs of the commissure to secure the pair of commissure tabs together; a second fastener that extends between an interior fold of a first folded commissure tab of the pair of commissure tabs and the corresponding attachment member to secure the first folded commissure tab to the attachment member; and a third fastener that extends between an interior fold of a second folded commissure tab of the pair of commissure tabs and the corresponding attachment member to secure the second folded commissure tab to the attachment member.

Example 70. The prosthetic heart valve of any example herein, particularly example 67, wherein the majority portions of each overlapping layer overlap in a radial direction, wherein the radial direction is relative to a central longitudinal axis of the prosthetic heart valve.

Example 71. The prosthetic heart valve of any example herein, particularly example 70, wherein the plurality of fasteners includes: a first fastener that is wrapped around at least a portion of the series of overlapping layers of both commissure tabs of the pair of commissure tabs of the commissure to secure the pair of commissure tabs together; a second fastener that extends from a first interior fold of the folded commissure tab, through two overlapping layers of the series of overlapping layers, and to the corresponding attachment member to secure an inner portion, relative to a lateral direction arranged tangent to a circumference of the annular frame, of the folded commissure tab to the attachment member; and a third fastener that extends from a second interior fold of the folded commissure tab, through one layer of the series of overlapping layers, and to the corresponding attachment member to secure an outer portion, relative to the lateral direction, of the folded commissure tab to the attachment member.

Example 72. The prosthetic heart valve of any example herein, particularly example 70, wherein the plurality of fasteners includes: a first fastener that extends through a first layer of the series of the overlapping layers and to the attachment member; a second fastener that extends through a second layer and third layer of the overlapping layers; and a third fastener that extends through each of the first layer, second layer, and third layer and to the attachment member.

In view of the many possible embodiments to which the principles of the disclosed technology may be applied, it should be recognized that the illustrated embodiments are only preferred examples of the disclosed technology and should not be taken as limiting the scope of the claimed subject matter. Rather, the scope of the claimed subject matter is defined by the following claims and their equivalents.

The application further comprises the following embodiments
1. A method of assembling a prosthetic heart valve comprising a plurality of leaflets, comprising:
   forming a plurality of commissures with the plurality of leaflets, wherein each commissure is formed by:
      folding each commissure tab of each leaflet of the plurality of leaflets into a series of overlapping layers, wherein each leaflet includes two opposing commissure tabs arranged on opposite sides of a body of the leaflet, the body configured to move
   during operation of the prosthetic heart valve, wherein each folded commissure tab is arranged outward of, in a lateral direction arranged tangent to a circumference of the prosthetic heart valve, a portion of the body of the leaflet that is directly connected to the commissure tab;
      pairing each folded commissure tab of each leaflet with a folded commissure tab of another leaflet; and
      securing at least a portion of the series of overlapping layers of each folded commissure tab in its folded configuration; and
   for each commissure, attaching the commissure to a respective commissure support portion of a frame of the prosthetic heart valve, either directly or via an attachment member configured to be coupled to the commissure support portion, wherein the body of each leaflet is separated from the commissure support portion, the attachment member, and each fastener of one or more fasteners used for the securing and attaching via the folded commissure tab.
2. The method of embodiment 1, wherein the folding includes folding each commissure tab so that a first portion of the commissure tab, the first portion directly connected to the body of the corresponding leaflet, extends radially outward from the body, a second portion is bent over the first portion and extends radially inward and toward the body, and a third portion is bent over the second portion and extends radially outward and away from the body, wherein a majority of each of the first, second, and third portions are arranged in parallel with one another and overlap in the lateral direction.
3. The method of embodiment 2, wherein the securing includes, for each pair of folded commissure tabs, wrapping a band around an outer edge of the second portion of a first folded commissure tab of the pair of folded commissure tabs to and around an outer edge of the second portion of a second folded commissure tab of the pair of commissure tabs, to secure the first and second folded commissure tabs to one another.
4. The method of either embodiment 2 or embodiment 3, wherein the attaching includes:
   for each folded commissure tab, extending a first fastener from an inner edge of a bend between the first portion and the second portion to the commissure support portion or the attachment member to secure the folded commissure tab to the commissure support portion or the attachment member; and
   securing a terminal, free end of each folded commissure tab to the commissure support portion or attachment member via a second fastener.
5. The method of embodiment 1, wherein the folding includes folding each commissure tab so that a first portion of the commissure tab, the first portion directly connected to the body of the corresponding leaflet, extends radially outward from the body, a second portion is bent away from the first portion and extends laterally outward, in the lateral direction, from the first portion, a third portion is folded over the second portion and extends laterally inward, and a fourth portion is folded over the third portion and extends laterally outward, wherein a majority of each of the second, third, and fourth portions are arranged in parallel with one another and overlap in a radial direction.
6. The method of embodiment 5, wherein the securing includes, for each pair of folded commissure tabs, wrapping a band around an inner edge of a bend between the third and fourth portions of a first folded commissure tab of the pair of folded commissure tabs to and around an inner edge of a bend between the third and fourth portions of a second folded commissure tab of the pair of commissure tabs, to secure the first and second folded commissure tabs to one another.
7. The method of either embodiment 5 or embodiment 6, wherein the attaching includes, for each folded commissure tab:
   extending a first fastener from an inner edge of a bend between the third portion and the fourth portion to the commissure support portion or the attachment member to secure the folded commissure tab to the commissure support portion or the attachment member;
   extending a second fastener from an inner edge of a bend between the second portion and the third portion to the commissure support portion or the attachment member to secure the folded commissure tab to the commissure support portion or the attachment member; and
   securing a terminal, free end of each folded commissure tab to the commissure support portion or the attachment member via a third fastener.
8. The method of any one of embodiments 5-7, further comprising arranging a fabric strip against an outer surface of the commissure tab in a region through which a fastener extends, the fabric strip arranged between an end of the fastener and the outer surface of the commissure tab.
9. The method of embodiment 1, wherein the folding includes folding each commissure tab so that a first portion of the commissure tab, the first portion directly connected to the body of the corresponding leaflet, extends radially outward, in the lateral direction, from the body, a second portion is bent away from the first portion and extends laterally outward from the first portion, a third portion is bent around from the second portion and extends laterally inward, and a fourth portion is folded over the third portion and extends laterally outward, wherein the fourth portion is arranged between the second and third portions, in the radial direction, and wherein a majority of each of the second, third, and fourth portions are arranged in parallel with one another and overlap in the radial direction.
10. The method of embodiment 9, wherein the securing and attaching includes, for each commissure tab, extending a first fastener from an inner edge of the second portion, through the second portion, and to the commissure support portion or attachment member, extending a second fastener from an outer edge of the third portion, through the third and fourth portions, and to an outer edge of the fourth portion, and extending a third fastener from an outer edge of the third portion, through each of the third portion, the fourth portion, and the second portion, and to the commissure support portion or attachment member.
11. The method of embodiment 10, wherein the securing and attaching via the first fastener and the second fastener occurs during the folding and the securing and attaching via the third fastener occurs after the folding is complete.
12. The method of any one of embodiments 1-11, wherein attaching the folded commissure tabs of the leaflet assembly to the commissure support portion of the prosthetic heart valve or the attachment member configured to be coupled to the commissure support portion includes attaching the folded commissure tabs of the leaflet assembly to the attachment member and further comprising securing the attachment member to the commissure support portion via an additional fastener.
13. The method of any one of embodiments 1-11, wherein attaching the commissure to the respective commissure support portion of the frame of the prosthetic heart valve includes attaching the commissure to a commissure support portion of an actuator or an expansion and locking mechanism of the frame.
14. A prosthetic heart valve, comprising:
   an annular frame comprising a plurality of commissure support portions;
   a plurality of attachment members, each secured to a corresponding support portion of the plurality of commissure support portions;
   a plurality of leaflets, each leaflet comprising a body and two opposing commissure tabs arranged on opposite sides of the body, wherein each commissure tab is folded into a series of overlapping layers including at least three layers, wherein majority portions of each overlapping layer are arranged in parallel with one another, wherein each folded commissure tab is arranged adjacent to another folded commissure tab of an adjacent leaflet to form a pair of commissure tabs forming a commissure, and wherein each commissure is coupled to a corresponding attachment member of the plurality of attachment members; and
   wherein, for each folded commissure tab of each commissure, a plurality of fasteners secure inner and outer portions, relative to a lateral direction arranged tangent to a circumference of the annular frame, of the folded commissure tab to the corresponding attachment member, wherein each of the plurality of fasteners are separated from the body of the leaflet to which the folded commissure tab is connected by folds of the folded commissure tab.
15. The prosthetic heart valve of embodiment 14, wherein the majority portions of each overlapping layer overlap in the lateral direction and wherein the plurality of fasteners includes:
   a first fastener that is wrapped around at least a portion of the series of overlapping layers of both commissure tabs of the pair of commissure tabs of the commissure to secure the pair of commissure tabs together;
   a second fastener that extends between an interior fold of a first folded commissure tab of the pair of commissure tabs and the corresponding attachment member to secure the first folded commissure tab to the attachment member; and
   a third fastener that extends between an interior fold of a second folded commissure tab of the pair of commissure tabs and the corresponding attachment member to secure the second folded commissure tab to the attachment member.
16. The prosthetic heart valve of embodiment 14, wherein the majority portions of each overlapping layer overlap in a radial direction, wherein the radial direction is relative to a central longitudinal axis of the prosthetic heart valve.
17. The prosthetic heart valve of embodiment 16, wherein the plurality of fasteners includes:
   a first fastener that is wrapped around at least a portion of the series of overlapping layers of both commissure tabs of the pair of commissure tabs of the commissure to secure the pair of commissure tabs together;
   a second fastener that extends from a first interior fold of the folded commissure tab, through two overlapping layers of the series of overlapping layers, and to the corresponding attachment member to secure an inner portion, relative to a lateral direction arranged tangent to a circumference of the annular frame, of the folded commissure tab to the attachment member; and
   a third fastener that extends from a second interior fold of the folded commissure tab, through one layer of the series of overlapping layers, and to the corresponding attachment member to secure an outer portion, relative to the lateral direction, of the folded commissure tab to the attachment member.
18. The prosthetic heart valve of embodiment 16, wherein the plurality of fasteners includes:
   a first fastener that extends through a first layer of the series of the overlapping layers and to the attachment member;
   a second fastener that extends through a second layer and third layer of the overlapping layers; and
   a third fastener that extends through each of the first layer, second layer, and third layer and to the attachment member.
19. A prosthetic heart valve, comprising:
   an annular frame comprising a plurality of commissure support portions;
   a plurality of attachment members, each secured to a corresponding support portion of the plurality of commissure support portions; and
   a plurality of leaflets, each leaflet comprising a body and two opposing commissure tabs arranged on opposite sides of the body, wherein each commissure tab is folded into a series of overlapping layers where majority portions of each overlapping layer are arranged in parallel with one another and overlap in a lateral direction, the lateral direction arranged tangent to a circumference of the annular frame, wherein each folded commissure tab is arranged adjacent to another folded commissure tab of an adjacent leaflet to form a pair of commissure tabs forming a commissure, and wherein each commissure is coupled to a corresponding attachment member of the plurality of attachment members; and
   wherein for each commissure:
      a first fastener is wrapped around at least a portion of the series of overlapping layers of both commissure tabs of the pair of commissure tabs of the commissure to secure the pair of commissure tabs together;
      a second fastener extends between an interior fold of a first folded commissure tab of the pair of commissure tabs and the corresponding attachment member to secure the first folded commissure tab to the attachment member;
      a third fastener extends between an interior fold of a second folded commissure tab of the pair of commissure tabs and the corresponding attachment member to secure the second folded commissure tab to the attachment member; and
      wherein each of the first, second, and third fasteners are separated from the bodies of the leaflets to which the commissure tabs of the pair of commissure tabs are connected by folds of the commissure tabs.
20. The prosthetic heart valve of embodiment 19, wherein the series of overlapping layers of each folded commissure tab comprises: a first portion that is directly connected to the body and extends radially outward from the body and toward the attachment member and commissure support portion to which the folded commissure tab is coupled, a second portion that is folded over the first portion and extends radially inward and toward the body, and a third portion that is folded over the second portion and extends radially outward and toward the attachment member and commissure support portion, wherein a majority of each of the first, second, and third portions are arranged in parallel with one another and overlap in the lateral direction.
21. The prosthetic heart valve of embodiment 20, wherein the first fastener is wrapped around the folded portions of the pair of commissure tabs of the commissure, from an outer edge of the second portion of a first folded commissure tab of the pair of commissure tabs to an outer edge of the second portion of a second folded commissure tab of the pair of commissure tabs, to secure the first and second portions of each folded commissure tab of the pair of commissure tabs together.
22. The prosthetic heart valve of either embodiment 20 or embodiment 21, wherein the second fastener extends from an inner edge of a bend between the first and second portions of the first folded commissure tab, through a thickness of the bend, and to the corresponding attachment member to secure the first folded commissure tab to the attachment member and wherein the third fastener extends from an inner edge of a bend between the first and second portion of the second folded commissure tab, through a thickness of the bend, and to the corresponding attachment member to secure the second folded commissure tab to the attachment member.
23. The prosthetic heart valve of any one of embodiments 19-22, wherein, for each commissure, an outermost, free end of the first folded commissure tab is secured to the attachment member via a fourth fastener and an outermost, free end of the second folded commissure tab is secured to the attachment member via a fifth fastener.

## Claims

1. A prosthetic heart valve (10) comprising a plurality of leaflets (304a, b) and prosthetic heart valves (10) including a plurality of leaflets (304a, b) assembled together to form commissures (24) configured to be coupled to a frame (12) of the prosthetic heart valve (10);
wherein the commissures (24) are formed by coupling a pair of adjacent commissure tabs (308a, b) of adjacent leaflets (304a, b) of the prosthetic heart valve (10) to one another;
wherein the commissures (24) include a pair of two commissure tabs (308a, b), each of the commissure tabs (308a, b) folded into a series of overlapping layers where majority portions of each overlapping layer are arranged in parallel with one another; and
wherein:
one or more fasteners (328a, b) used to secure the two commissure tabs (308a, b) of the commissure (24) to one another and/or to the commissure support portion (318), or
an attachment member (316) configured to be coupled to the commissure support portion (318);
are separated from movable regions of bodies of the leaflets (304a, b) to which the commissure tabs (308a, b) of the pair of commissure tabs (308a, b) are connected by folds of the commissure tabs (308a, b).

2. The prosthetic valve of claim 1, wherein each folded commissure tab (308a, b) is arranged outward of, in a lateral direction that is arranged tangent to a circumference of the frame (12) of the prosthetic heart valve (10), a portion of the body (306a, b) of the leaflet (304a, b) that is directly connected to the commissure tab (308a, b).

3. The prosthetic valve of any one of the preceding claims, wherein the body (306a, b) of each leaflet (304a, b) is separated from each of the commissure support portion (318), the attachment member (316), and the one or more fasteners (328a, b), by the folded commissure tab (308a, b).

4. The prosthetic valve of any one of the preceding claims, wherein the folded commissure tabs (308a, b) are configured to offset bending regions of the leaflets (304a, b) away from the commissure support portions (318) of the frame (12) or attachment members (316) attached thereto.

5. The prosthetic valve of any one of the preceding claims, wherein the fasteners (328a, b) used to attach the commissure (24) to the attachment member (316) and/or to the commissure support portion (318) extend through the tab portions and their folds, and do not penetrate the main bodies (306a, b) of the leaflets (304a, b), including their movable or bendable regions.

6. The prosthetic valve of any one of the preceding claims, configured to be implanted in the native aortic annulus.

7. The prosthetic valve of any one of the preceding claims, wherein the majority portions of each overlapping layer overlap in a radial direction, wherein the radial direction is relative to a central longitudinal axis of the prosthetic heart valve (10).

8. The prosthetic valve of claim 7, wherein the one or more fasteners (328a, b) include:
a first fastener (328a, b) that is wrapped around at least a portion of the series of overlapping layers of both commissure tabs (308a, b) of the pair of commissure tabs (308a, b) of the commissure (24) to secure the pair of commissure tabs (308a, b) together;
a second fastener (330a, b) that extends from a first interior fold of the folded commissure tab (308a, b), through two overlapping layers of the series of overlapping layers, and to the corresponding attachment member (316) to secure an inner portion, relative to a lateral direction arranged tangent to a circumference of the annular frame (12), of the folded commissure tab (308a, b) to the attachment member (316); and
a third fastener (534a, b) that extends from a second interior fold of the folded commissure tab (308a, b), through one layer of the series of overlapping layers, and to the corresponding attachment member (316) to secure an outer portion, relative to the lateral direction, of the folded commissure tab (308a, b) to the attachment member (316).

9. The prosthetic valve of claim 7, wherein the one or more fasteners (328a, b) include:
a first fastener (328a, b) that extends through a first layer of the series of the overlapping layers and to the attachment member (316);
a second fastener (330a, b) that extends through a second layer and third layer of the overlapping layers; and
a third fastener (534a, b) that extends through each of the first layer, second layer, and third layer and to the attachment member (316).

10. The prosthetic heart valve (10) of any one of claims 1 to 6, wherein the majority portions of each overlapping layer overlap in the lateral direction.

11. The prosthetic valve of claim 10, wherein wherein the one or more fasteners (328a, b) include:
a first fastener (328a, b) that is wrapped around at least a portion of the series of overlapping layers of both commissure tabs (308a, b) of the pair of commissure tabs (308a, b) of the commissure (24) to secure the pair of commissure tabs (308a, b) together;
a second fastener (330a, b) that extends between an interior fold of a first folded commissure tab (308a, b) of the pair of commissure tabs (308a, b) and the corresponding attachment member (316) to secure the first folded commissure tab (308a, b) to the attachment member (316); and
a third fastener (534a, b) that extends between an interior fold of a second folded commissure tab (308a, b) of the pair of commissure tabs (308a, b) and the corresponding attachment member (316) to secure the second folded commissure tab (308a, b) to the attachment member (316).

12. The prosthetic valve of any one of claims 8 to 11, wherein the first fastener (328a, b) is a suture.

13. The prosthetic valve of any one of claims 8 to 12, wherein the second fastener (330a, b) is a suture.

14. The prosthetic valve of any one of claims 8 to 13, wherein the third fastener (534a, b) is a suture.

15. The prosthetic valve of any one of the preceding claims, wherein the fasteners (328a, b) are arranged within the folds and do not contact the main bodies (306a, b) of the leaflets (304a, b), even during operation of the prosthetic valve.
